(19) Europäisches Patentamt / European Patent Office / Office européen des brevets

(11) **EP 3 799 875 A1**

(12) **DEMANDE DE BREVET EUROPEEN**

(43) Date de publication:
**07.04.2021 Bulletin 2021/14**

(21) Numéro de dépôt: **19209272.4**

(22) Date de dépôt: **23.12.2014**

(51) Int Cl.:
*A61K 31/728* (2006.01)     *A61K 31/4458* (2006.01)
*A61K 9/00* (2006.01)     *A61K 9/107* (2006.01)
*A61K 47/10* (2017.01)     *A61K 8/49* (2006.01)
*A61K 8/73* (2006.01)     *A61K 8/04* (2006.01)
*A61Q 19/08* (2006.01)     *A61P 19/02* (2006.01)
*A61P 17/00* (2006.01)     *A61P 23/00* (2006.01)
*A61P 43/00* (2006.01)

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **23.12.2013 FR 1363505**

(62) Numéro(s) de document de la (des) demande(s)
initiale(s) en application de l'article 76 CBE:
**17150080.4 / 3 173 086**
**14827785.8 / 3 049 091**

(27) Demande déposée antérieurement:
**23.12.2014 PCT/EP2014/079270**

(71) Demandeur: **Laboratoires Vivacy
75116 Paris (FR)**

(72) Inventeurs:
- **BON BETEMPS, Jérémie
  73410 ALBENS (FR)**
- **VITALLY, Guy
  73370 LE BOURGET-DU-LAC (FR)**

(74) Mandataire: **Tripoz, Inès et al
Cabinet Tripoz
Le Pôle Sud
22 rue Seguin
69002 Lyon (FR)**

Remarques:
•Cette demande a été déposée le 14.11.2019 comme demande divisionnaire de la demande mentionnée sous le code INID 62.
•Les revendications 16-20 sont réputées abandonnées pour défaut de paiement des taxes de revendication (règle 45(3) CBE).

(54) **COMPOSITIONS D'ACIDE HYALURONIQUE COMPRENANT DE LA MÉPIVACAÏNE**

(57) L'invention concerne donc une composition aqueuse stérilisée, comprenant au moins un acide hyaluronique et au moins de la mépivacaïne et éventuellement un ou plusieurs composés additionnels, le ratio massique entre la concentration en acide hyaluronique [HA] et la concentration en mépivacaïne [MEPI] : [HA]/[MEPI] étant supérieur ou égal à 0,1 ; [HA]/[MEPI] ≥ 0,1.

L'invention concerne également l'utilisation de la mépivacaïne en substitution de la lidocaïne en quantité équivalente pour obtenir une composition d'acide hyaluronique comprenant un anesthésique local dont les propriétés rhéologiques après stérilisation à la chaleur sont supérieures aux propriétés rhéologiques d'une même composition d'acide hyaluronique comprenant de la lidocaïne.

EP 3 799 875 A1

**Description**

L'invention concerne le domaine des gels et hydrogels biodégradables utilisés en tant que biomatériaux et plus particulièrement dans les domaines médicaux et esthétiques.

Parmi les applications médicales on citera par exemple les injections pour remplacer les liquides biologiques déficients par exemple dans les articulations pour remplacer le liquide synovial, l'injection suite à une chirurgie pour éviter les adhésions péritonéales, les injections périurétrales pour traiter l'incontinence et les injections suite à une chirurgie de la presbytie.

Parmi les applications esthétiques on citera par exemple les injections pour le comblement des rides, des ridules et des défauts cutanés ou l'augmentation des volumes par exemple les lèvres, les pommettes, etc.

Dans toutes ces applications les gels et hydrogels utilisés doivent présenter des propriétés optimisées en termes de rémanence in vivo, de rhéologie et de viscosité pour garantir une bonne injectabilité, ces hydrogels étant injectés à l'aide d'aiguilles qui doivent rester les plus fines possible, pour garantir la précision des gestes des praticiens et minimiser les réactions post injections.

Les gels et hydrogels utilisés sont à base de polymères qui sont choisis parmi les polysaccharides comme l'acide hyaluronique, le kératane, l'héparine, la cellulose et les dérivés de cellulose, l'acide alginique, le xanthane, la carraghénane, le chitosane, la chondroïtine et leurs sels biologiquement acceptables.

Pour améliorer ces gels et/ou hydrogels et/ou leur conférer des propriétés particulières, un certain nombre d'additifs peuvent leur être ajoutés.

L'un des inconvénients principal de l'addition d'additifs est la dégradation potentielle des propriétés rhéologiques et/ou viscoélastiques des gels finaux ou de leur stabilité, soit directement lors de l'addition soit lors des phases de stérilisation, soit dans le temps par exemple lors du stockage.

L'article de Michael H Gold (Clinical Interventions in Aging, 2007, 369-376) retrace rapidement l'historique de l'évolution des produits de comblement dermique. Les premières compositions développées dans ce but étaient à base de collagène. Les produits Zyderm® (approuvé par la FDA en 1981) et Zyplast® (approuvé par la FDA en 1985) étaient à base de collagène issu de bovin. Par la suite deux produits similaires, mais à base de collagène d'origine humaine ont été développés (CosmoDerm® and CosmoPlast® approuvés par la FDA en 2003).

A la fin des années 80 Balazs a développé la première composition pour le comblement dermique à base d'acide hyaluronique. Depuis des améliorations ont été apportées afin d'augmenter la stabilité des compositions à base d'acide hyaluronique.

Comme indiqué dans l'article de Gold cité ci-dessus, les compositions à base de collagène contenaient de la lidocaïne pour atténuer la douleur associée à la technique d'injection. Cependant, dans un premier temps, les compositions à base d'acide hyaluronique ne contenaient pas d'anesthésique local en raison des problèmes de stabilité dus aux additifs comme exposé ci-dessus.

Depuis quelques années des efforts ont été fournis dans l'optique d'incorporer un anesthésique local, en particulier la lidocaïne dans des gels à base d'acide hyaluronique tout en garantissant une certaine stabilité. Puragen™ Plus commercialisé par Mentor Corporation est d'après l'article de Gold la première composition de comblement à base d'acide hyaluronique comprenant de la lidocaïne. La demande de brevet WO 2005/112888 au nom de Mentor Corporation publiée le 1 Décembre 2005 décrit une méthode de préparation d'hydrogels injectable pouvant comprendre de la lidocaïne.

De nombreuses demandes de brevet portant sur des compositions à base d'acide hyaluronique et comprenant de la lidocaïne ont été déposées par les acteurs du domaine.

La demande de brevet WO 2005/067994 au nom d'Anika Therapeutics publiée le 28 Juillet 2005 décrit à l'exemple 21 des compositions à base de particules de gels d'acide hyaluronique réticulés comprenant de la lidocaïne. La lidocaïne est le seul anesthésique local exemplifié.

La demande de brevet WO 2010/015901 au nom d'Allergan publiée le 11 février 2010 décrit des compositions injectables à base d'acide hyaluronique pour le comblement dermique comprenant de la lidocaïne. Dans ce document, seules des compositions à base de lidocaïne sont exemplifiées.

La demande de brevet WO 2010/052430 au nom d'Anteis publiée le 14 Mai 2010 décrit des compositions à base d'acide hyaluronique comprenant de la lidocaïne et un ou plusieurs polyol(s).

La demande de brevet WO 2012/104419 au nom de Q-MED AB publiée le 9 Août 2012 décrit également des compositions injectables à base d'acide hyaluronique pour le comblement dermiques comprenant un anesthésique local. En particulier, des compositions comprenant de la lidocaïne, de la bupivacaïne (pka = 8,1) et de la tetracaïne (pka = 8,5) sont exemplifiées. Les anesthésiques locaux préférés dans la description sont la bupivacaine, la lidocaine et la ropivacaine (pka = 8,1). Aucune composition incorporant un anesthésique local dont le pka est inférieur à celui de la lidocaïne n'a donc été exemplifié ni cité individuellement. L'essentiel des exemples décrits dans la demande de brevet WO 2012/104419 concerne des compositions comprenant de la lidocaïne.

La demande WO 2013/186493 divulgue des compostions d'acide hyaluronique incluant un sucrose octasulfate.

Aucune formulation comprenant un anesthésique local n'est exemplifié et tous les exemples illustrent des compositions subissant une stérilisation terminale par autoclavage.

La demande FR 2 979 539 au nom de TEOXANE décrit des formaulations comprenant un anesthésique local et d'autres actifs, là encore seules des compositions comprenant de la lidocaïne sont décrites.

La demande CN 102805882 est relative à des compositions d'acide hyaluronique dans lesquelles sont ajoutés, juste avant leur utilisation, des anesthésiques locaux, mais aucun exemple n'est décrit.

La demande WO 2013/186493 divulgue des compositions d'acide hyaluronique incluant un dérivé de vitamine C. Aucune formulation comprenant un anesthésique local n'est exemplifiée.

La demande KR20140025117 décrit des compositions à base d'acide hyaluronique compreant des anesthésiques et seules des compositions comprenant de la lidocaïne sont décrites.

D'autres anesthésiques locaux sont cités dans la littérature, mais rarement exemplifiés et seuls des produits comprenant de la lidocaïne sont à ce jour, commercialisés.

Une demande de brevet EP 2 581 079 au nom de Biopolymer GmbH & Co. KG décrit des compositions à base d'acide hyaluronique et de prilocaïne, présentant un profil de relargage rapide de la prilocaïne.

En effet une des améliorations restant à atteindre est d'obtenir une action des anesthésiques locaux la plus rapide possible.

En dépit de tout l'art antérieur existant relatif à des compositions d'acide hyaluronique comprenant un anesthésique local, quasiment tous les exemples de l'art antérieur sont relatifs à la lidocaïne et aucun exemple de l'art antérieur n'est relatif à la mépivacaïne.

Parmi les candidats potentiels, on trouve les anesthésiques locaux de type aminoamides à action rapide, dont le groupe est constitué de la lidocaïne, l'étidocaïne, la mépivacaïne, la prilocaïne et l'articaïne.

Le délai d'action de ces anesthésiques locaux dépend de leur pKa qui sont compris entre 7,7 et 8,0. A pH physiologique, l'anesthésique local ayant le délai d'action le plus rapide est celui dont le pKa sera le plus proche de 7,4, car sa forme basique, non ionisée liposoluble, sera celle qui va pénétrer l'épinérium et la membrane neuronale, permettant par la suite à la molécule d'être plus rapidement disponible pour bloquer les canaux à sodium. Parmi les candidats possibles de type aminoamide à action rapide, la mépivacaïne est l'anesthésique local qui présente le pKa le plus faible du groupe car son pKa est de 7,7, la mépivacaïne a donc, en théorie, le délai d'action le plus rapide du groupe.

Cependant un des risques présentés par l'incorporation de molécules de ce type est leur tendance à la précipitation. En effet, la forme basique est liposoluble, ainsi lors de leur incorporation dans le gel aqueux, en général formulé à un pH proche du pH physiologique, c'est à dire 7,4, l'anesthésique aura une forte propension à précipiter.

La précipitation des anesthésiques locaux est assez difficile à appréhender. Le plus souvent, le pKa est considéré comme un bon indicateur de précipitation : plus le pKa est faible, plus les risques de précipitations sont élevés (toutes conditions étant égales par ailleurs). Ainsi, la mépivacaïne est, vu son pKa de 7,7, le plus mauvais candidat du groupe des anesthésiques locaux du groupe aminoamide à action rapide du point de vue du pKa. C'est sans doute pour cette raison que la mépivacaïne n'a jamais été exemplifiée dans l'art antérieur. En effet, même dans des demandes investiguant sur les possibilités d'incorporation d'anesthésiques locaux alternatifs à la lidocaïne comme par exemple la demande WO 2012/104419 au nom de Q-MED AB, la mépivacaïne est citée, mais seuls des anesthésiques locaux alternatifs à la lidocaïne ayant un pKa plus élevés sont exemplifiés : bupivacaine (pKa 8,1), tétracaïne (pka 8,5). Enfin, la prilocaïne, envisagée dans demande de brevet EP 2 581 079 au nom de Biopolymer GmbH & Co. KG, a un pKa de 7,9.

En effet, il est impératif qu'aucune précipitation n'intervienne dans les gels qui sont injectés avec de fines aiguilles dans le but de corriger les rides. L'utilisation en esthétique impose que rien ne puisse entraver l'injection, sous peine de mauvaise application et donc de défaut dans le comblement. De plus, un précipité provoquerait les mêmes effets qu'un corps étranger et donc entrainerait des risques d"inflammation. Par ailleurs, la formation d'un précipité diminuerait la quantité d'anesthésique local en solution et par conséquent diminuerait sa biodisponibilité et donc son efficacité.

Sans doute à cause des inconvénients précités, bien que listée parmi les anesthésiques locaux pouvant être incorporés au sein de compositions à base d'acide hyaluronique notamment dans les demandes de brevets WO 2010/015901 et WO 2012/104419, aucun exemple de gel à base d'acide hyaluronique comprenant de la mépivacaïne n'a été décrit.

On connaît de la littérature, un article de Cho et al, Pak. J. Pharm. Sci., 2001 Jan ; 24(1) : 87-93 qui décrit des études de relargage de la mépivacaïne à partir de gels d'hydroxypropyl methylcellulose (HPMC). Ces compositions sont formulées sous forme de gel pour une application directe sur la peau et une administration transdermique. Il est décrit dans cet article que l'augmentation de la concentration de mépivacaïne et l'augmentation de la température augmente le taux de relargage de la mépivacaïne.

Aucune formulation d'acide hyaluronique comprenant de la mépivacaïne n'a été à ce jour décrite, sans doute à cause des difficultés potentielles à la formuler à pH physiologique.

De façon surprenante, la demanderesse a montré que l'incorporation de mépivacaïne dans des gels à base d'acide hyaluronique permettait d'une part d'obtenir des compositions à pH proche du pH physiologique sans précipité et ce malgré le pKa défavorable de la mépivacaïne et d'autre part que ces compositions stérilisées présentaient des propriétés rhéologiques moins altérées lors de leur stérilisation par rapport à des compositions comprenant un autre anesthésique local du même groupe.

De plus cette moindre altération de la composante élastique G' lors de la stérilisation a été observée quels que soient les éventuels autres excipients ou composés additionnels classiquement utilisés dans la formulation de gels de comblement.

De façon surprenant également l'addition de mépivacaïne permet d'obtenir des compositions en présence de polyols qui ont systématiquement des propriétés rhéologiques améliorées par rapport à des compositions ne comprenant ni polyol, ni anesthésique.

L'invention concerne donc une composition aqueuse stérilisée à pH proche du pH physiologique, comprenant au moins un acide hyaluronique et au moins de la mépivacaïne, le ratio massique entre la concentration en acide hyaluronique [HA] et la concentration en mépivacaïne [MEPI] : [HA]/[MEPI] étant supérieur ou égal à 0,1 ; [HA]/[MEPI] $\geq$ 0,1.

On entend par acide hyaluronique, l'acide hyaluronique, réticulé ou non réticulé, seul ou en mélange, éventuellement modifié chimiquement par substitution, seul ou en mélange, éventuellement sous forme de l'un de ses sels, seul ou en mélange.

On entend par mépivacaïne, la mépivacaïne ou l'un de ses sels, seuls ou en mélange.

Dans un mode de réalisation, le ratio massique entre la concentration en acide hyaluronique [HA] et la concentration en mépivacaïne [MEPI] : [HA]/[MEPI] est compris entre 0,1 et 50, 0,1 $\leq$ [HA]/[MEPI] $\leq$ 50.

Dans un mode de réalisation, le ratio massique entre la concentration en acide hyaluronique [HA] et la concentration en mépivacaïne [MEPI] : [HA]/[MEPI] est compris entre 0,5 et 40, 0,5 $\leq$ [HA]/[MEPI] $\leq$ 40.

Dans un mode de réalisation, le ratio massique entre la concentration en acide hyaluronique [HA] et la concentration en mépivacaïne [MEPI] : [HA]/[MEPI] est compris entre 1 et 30, 1 $\leq$ [HA]/[MEPI] $\leq$ 30.

Dans un mode de réalisation, le ratio massique entre la concentration en acide hyaluronique [HA] et la concentration en mépivacaïne [MEPI] : [HA]/[MEPI] est compris entre 2 et 20, 2 $\leq$ [HA]/[MEPI] $\leq$ 20.

Dans un mode de réalisation, le ratio massique entre la concentration en acide hyaluronique [HA] et la concentration en mépivacaïne [MEPI], [HA]/[MEPI] est compris entre 7/3 et 26/3, 7/3 $\leq$ [HA]/[MEPI] $\leq$ 26/3.

Dans un mode de réalisation, le ratio massique entre la concentration en acide hyaluronique [HA] et la concentration en mépivacaïne [MEPI], [HA]/[MEPI] est compris entre 2 et 20/3, 2 $\leq$ [HA]/[MEPI] $\leq$ 20/3.

Dans un mode de réalisation, le ratio massique entre la concentration en acide hyaluronique [HA] et la concentration en mépivacaïne [MEPI], [HA]/[MEPI] est compris entre 2 et 10/3, 2 $\leq$ [HA]/[MEPI] $\leq$ 10/3.

Dans un mode de réalisation, le ratio massique entre la concentration en acide hyaluronique [HA] et la concentration en mépivacaïne [MEPI], [HA]/[MEPI] est de 20.

Dans un mode de réalisation, le ratio massique entre la concentration en acide hyaluronique [HA] et la concentration en mépivacaïne [MEPI], [HA]/[MEPI] est de 26/3.

Dans un mode de réalisation, le ratio massique entre la concentration en acide hyaluronique [HA] et la concentration en mépivacaïne [MEPI], [HA]/[MEPI] est de 20/3.

Dans un mode de réalisation, le ratio massique entre la concentration en acide hyaluronique [HA] et la concentration en mépivacaïne [MEPI], [HA]/[MEPI] est de 10/3.

Dans un mode de réalisation, le ratio massique entre la concentration en acide hyaluronique [HA] et la concentration en mépivacaïne [MEPI], [HA]/[MEPI] est de 7/3.

Dans un mode de réalisation, le ratio massique entre la concentration en acide hyaluronique [HA] et la concentration en mépivacaïne [MEPI], [HA]/[MEPI] est de 2.

Dans un mode de réalisation, la concentration en mépivacaïne [MEPI] est comprise entre 0,01 mg/g et 50 mg/g de poids total de ladite composition.

Dans un mode de réalisation, la concentration en mépivacaïne [MEPI] est comprise entre 0,05 mg/g et 45 mg/g de poids total de ladite composition.

Dans un mode de réalisation, la concentration en mépivacaïne [MEPI] est comprise entre 0,1 mg/g et 40 mg/g de poids total de ladite composition.

Dans un mode de réalisation, la concentration en mépivacaïne [MEPI] est comprise entre 0,2 mg/g et 30 mg/g de poids total de ladite composition.

Dans un mode de réalisation, la concentration en mépivacaïne [MEPI] est comprise entre 0,5 mg/g et 20 mg/g de poids total de ladite composition.

Dans un mode de réalisation, la concentration en mépivacaïne [MEPI] est comprise entre 1 mg/g et 15 mg/g de poids total de ladite composition.

Dans un mode de réalisation, la concentration en mépivacaïne [MEPI] est comprise entre 1 mg/g et 10 mg/g de poids total de ladite composition.

Dans un mode de réalisation, la concentration en mépivacaïne [MEPI] est comprise entre 1 mg/g et 6 mg/g de poids total de ladite composition.

Dans un mode de réalisation, la concentration en mépivacaïne [MEPI] est comprise entre 1 mg/g et 5 mg/g de poids total de ladite composition.

Dans un mode de réalisation, la concentration en mépivacaïne [MEPI] est comprise entre 2 mg/g et 5 mg/g de poids total de ladite composition.

Dans un mode de réalisation, la concentration en mépivacaïne [MEPI] est comprise entre 6 mg/g et 10 mg/g de poids total de ladite composition.

Dans un mode de réalisation, la concentration en mépivacaïne [MEPI] est de 1 mg/g de poids total de ladite composition.

Dans un mode de réalisation, la concentration en mépivacaïne [MEPI] est de 3 mg/g de poids total de ladite composition.

Dans un mode de réalisation, la concentration en mépivacaïne [MEPI] est de 4 mg/g de poids total de ladite composition.

Dans un mode de réalisation, la concentration en mépivacaïne [MEPI] est de 5 mg/g de poids total de ladite composition.

Dans un mode de réalisation, la concentration en mépivacaïne [MEPI] est de 6 mg/g de poids total de ladite composition.

Dans un mode de réalisation, la concentration en mépivacaïne [MEPI] est de 10 mg/g de poids total de ladite composition.

Dans un mode de réalisation, la mépivacaïne est choisie dans le groupe comprenant la mépivacaïne ou l'un de ses sels pharmaceutiquement acceptables.

Dans un mode de réalisation, la mépivacaïne est choisie dans le groupe constitué par l'hydrochlorure de mépivacaïne racémique, la mépivacaïne racémique, l'hydrochlorure de (R)-mépivacaïne, l'hydrochlorure de (S)-mépivacaïne, la (R)-mépivacaïne et la (S)-mépivacaïne ou l'un de leurs sels pharmaceutiquement acceptables.

Dans un mode de réalisation, la mépivacaïne est l'hydrochlorure de mépivacaïne racémique.

Dans un mode de réalisation, la mépivacaïne est l'hydrochlorure de (R)-mépivacaïne.

Dans un mode de réalisation, la mépivacaïne est l'hydrochlorure de (S)-mépivacaïne.

Dans un mode de réalisation, la mépivacaïne est la mépivacaïne racémique.

Dans un mode de réalisation, la mépivacaïne est la (R)-mépivacaïne.

Dans un mode de réalisation, la mépivacaïne est la (S)-mépivacaïne.

Dans un mode de réalisation, la concentration en acide hyaluronique [HA] est comprise entre 2 mg/g et 50 mg/g de poids total de ladite composition.

Dans un mode de réalisation, la concentration en acide hyaluronique [HA] est comprise entre 4 mg/g et 40 mg/g de poids total de ladite composition.

Dans un mode de réalisation, la concentration en acide hyaluronique [HA] est comprise entre 5 mg/g et 30 mg/g de poids total de ladite composition.

Dans un mode de réalisation, la concentration en acide hyaluronique [HA] est comprise entre 10 mg/g et 30 mg/g de poids total de ladite composition.

Dans un mode de réalisation, la concentration en acide hyaluronique [HA] est de 20 mg/g de poids total de ladite composition.

Dans un mode de réalisation, la composition aqueuse stérilisée selon l'invention est caractérisée en ce que la teneur totale en acide hyaluronique est comprise entre 0,2 et 5% en poids par rapport au poids total de ladite composition.

Dans un mode de réalisation, la composition aqueuse stérilisée selon l'invention est caractérisée en ce que la teneur totale en acide hyaluronique est supérieure ou égale à 1 % en poids par rapport au poids total de ladite composition.

Dans un mode de réalisation, la composition aqueuse stérilisée selon l'invention comprend au moins un acide hyaluronique non réticulé ou l'un de ses sels, seul ou en mélange.

Dans un mode de réalisation, la composition aqueuse stérilisée selon l'invention comprend au moins un acide hyaluronique réticulé ou l'un de ses sels, seul ou en mélange.

Dans un mode de réalisation, la composition aqueuse stérilisée selon l'invention comprend au moins un acide hyaluronique co-réticulé ou l'un de ses sels, seul ou en mélange.

Dans un mode de réalisation, la composition aqueuse stérilisée selon l'invention comprend au moins un acide hyaluronique modifié chimiquement par substitution, réticulé ou non réticulé, ou l'un de ses sels, seul ou en mélange.

Dans un mode de réalisation, l'acide hyaluronique est doublement réticulé tel que décrit dans la demande de brevet WO2000/046253 au nom de Fermentech Medical Limited.

Dans un mode de réalisation, la composition aqueuse stérilisée selon l'invention comprend un mélange d'acides hyaluroniques, ou l'un de leurs sels, réticulé et non réticulé.

Dans un mode de réalisation, la composition aqueuse stérilisée selon l'invention comprend un mélange d'acides hyaluroniques, ou l'un de leurs sels, réticulés.

Dans un mode de réalisation, le mélange d'acides hyaluroniques, ou l'un de leurs sels, réticulés est un mélange monophasique tel que celui décrit dans la demande de brevet WO2009/071697 au nom de la demanderesse.

Dans un mode de réalisation, le mélange d'acides hyaluroniques, ou l'un de leurs sels, réticulés est un mélange obtenu par mélange de plusieurs acides hyaluroniques, ou l'un de leurs sels, de masses moléculaires différentes préalablement à leur réticulation, tel que décrit dans la demande de brevet WO2004/092222 au nom de Cornéal Industrie.

Dans un mode de réalisation, la composition aqueuse stérilisée selon l'invention comprend au moins un acide hyaluronique, ou l'un de ses sels, substitué par un groupement apportant des propriétés lipophile ou hydratante, comme par exemple les acides hyaluroniques substitués tels que décrits dans la demande de brevet FR 2 983 483 au nom de la demanderesse.

Dans un mode de réalisation, l'acide hyaluronique est sous forme de sel de sodium ou de potassium.

On appelle Mw ou « masse moléculaire », la masse moléculaire moyenne en poids des polymères, mesurée en Daltons.

Dans un mode de réalisation, la composition selon l'invention est caractérisée en ce que la masse moléculaire Mw de l'au moins un acide hyaluronique est comprise dans un intervalle de 0,01 MDa et 5 MDa.

Dans un mode de réalisation, la composition selon l'invention est caractérisée en ce que la masse moléculaire Mw de l'au moins un acide hyaluronique est comprise dans un intervalle de 0,1 MDa et 3,5 MDa.

Dans un mode de réalisation, la composition selon l'invention est caractérisée en ce que la masse moléculaire Mw de l'au moins un acide hyaluronique est comprise dans un intervalle de 1 MDa et 3 MDa.

Dans un mode de réalisation, la composition selon l'invention est caractérisée en ce que la masse moléculaire Mw de l'au moins un acide hyaluronique est de 1 MDa .

Dans un mode de réalisation, la composition selon l'invention est caractérisée en ce que la masse moléculaire Mw de l'au moins un acide hyaluronique est de 3 MDa.

Dans la présente invention, le taux de réticulation X, est défini comme étant égal au rapport :

$$X = \frac{(\text{Nombre de moles de réticulant introduites dans le milieu réactionnel})}{(\text{Nombre de moles de motif dissacharidique introduites dans le milieu réactionnel})}$$

Dans un mode de réalisation, l'acide hyaluronique réticulé présente un taux de réticulation X compris entre 0,001 et 0,5.

Dans un mode de réalisation, l'acide hyaluronique réticulé présente un taux de réticulation X compris entre 0,01 et 0,4.

Dans un mode de réalisation, l'acide hyaluronique réticulé présente un taux de réticulation X compris entre 0,1 et 0,3.

Dans un mode de réalisation, l'acide hyaluronique réticulé présente un taux de réticulation X de 0,06.

Dans un mode de réalisation, l'acide hyaluronique réticulé présente un taux de réticulation X de 0,07.

Dans un mode de réalisation, l'acide hyaluronique réticulé présente un taux de réticulation X de 0,12.

Dans un mode de réalisation, la composition aqueuse stérilisée selon l'invention comprend, en outre, un autre polysaccharide.

Dans un mode de réalisation, cet autre polysaccharide est choisi dans le groupe constitué par la cellulose et ses dérivés et/ou l'acide alginique ou l'un de leurs sels.

La composition aqueuse est stérilisée, c'est-à-dire qu'elle subit après sa préparation une étape de stérilisation, ladite étape de stérilisation étant effectuée par la chaleur, la chaleur humide, le rayonnement gamma(y), ou par faisceau d'électrons accélérés (Electron-beam).

Dans un mode de réalisation, l'étape de stérilisation est réalisée par autoclavage à la vapeur.

Dans un mode de réalisation, la stérilisation par autoclavage à la vapeur est réalisé à une température de 121 à 134°C, pendant une durée adaptée à la température.

Par exemple la stérilisation par autoclavage à la vapeur est réalisée à une température comprise entre 127 et 130°C pendant une durée comprise entre 1 et 20 min.

Dans un mode de réalisation, l'étape de stérilisation est réalisée par irradiation par rayonnements gamma ($\gamma$).

Dans un mode de réalisation, la composition aqueuse stérilisée selon l'invention comprend en outre au moins un antioxydant.

L'invention concerne donc également une composition aqueuse stérilisée, comprenant au moins un acide hyaluronique, au moins de la mépivacaïne et au moins un antioxydant, le ratio massique entre la concentration en acide hyaluronique [HA] et la concentration en mépivacaïne [MEPI] : [HA]/[MEPI] étant supérieur à 0,1 ; $0,1 \le$ [HA]/[MEPI].

Dans un mode de réalisation, la composition aqueuse stérilisée selon l'invention est caractérisée en ce que le au moins antioxydant est choisi parmi les polyols.

Dans un mode de réalisation, la composition aqueuse stérilisée selon l'invention est caractérisée en ce que les polyols sont choisis dans le groupe constitué par le glycérol, le sorbitol, le propylène glycol, le xylitol, le mannitol, l'érythritol, le maltitol et le lactitol, seul ou en mélange.

Dans un mode de réalisation, la composition aqueuse stérilisée selon l'invention est caractérisée en ce que les polyols sont choisis dans le groupe constitué par le mannitol, le sorbitol, le maltitol et le glycérol, seul ou en mélange.

Dans un mode de réalisation, la composition aqueuse stérilisée selon l'invention est caractérisée en ce que les polyols sont choisis dans le groupe constitué par le mannitol et le sorbitol, seul ou en mélange.

Dans un mode de réalisation, la composition aqueuse stérilisée selon l'invention est caractérisée en ce que le polyol est le mannitol.

Dans un mode de réalisation, la composition aqueuse stérilisée selon l'invention est caractérisée en ce que le polyol est le sorbitol.

Dans un mode de réalisation, la composition aqueuse stérilisée selon l'invention est caractérisée en ce que le polyol est le maltitol.

Dans un mode de réalisation, la composition aqueuse stérilisée selon l'invention est caractérisée en ce que le polyol est le glycérol.

Dans un mode de réalisation, la composition aqueuse stérilisée selon l'invention est caractérisée en ce que l'antioxydant est un mélange de mannitol et de sorbitol.

De manière générale, le mannitol, de même que le sorbitol, seuls ou en mélange :

- procurent une bonne résistance à la dégradation par la stérilisation à la vapeur ;
- ont un fort pouvoir antioxydant ;
- sont facilement solubilisés dans des compositions d'acide hyaluronique.

Dans un mode de réalisation, la composition aqueuse stérilisée selon l'invention est caractérisée en ce que la teneur en polyol est comprise entre 10 et 40 mg/g en poids total de ladite composition.

Dans un mode de réalisation, la composition aqueuse stérilisée selon l'invention est caractérisée en ce que la teneur en polyol est comprise entre 15 et 30 mg/g en poids total de ladite composition.

Dans un mode de réalisation, la composition aqueuse stérilisée selon l'invention est caractérisée en ce que la teneur en polyol est comprise entre 15 et 25 mg/g en poids total de ladite composition.

Dans un mode de réalisation, la composition aqueuse stérilisée selon l'invention est caractérisée en ce que la teneur en polyol est comprise entre 20 et 40 mg/g en poids total de ladite composition.

Dans un mode de réalisation, la composition aqueuse stérilisée selon l'invention est caractérisée en ce que la teneur en polyol est comprise entre 20 et 30 mg/g en poids total de ladite composition.

Dans un mode de réalisation, la composition aqueuse stérilisée selon l'invention est caractérisée en ce que la teneur en polyol est comprise entre 25 et 35 mg/g en poids total de ladite composition.

Dans un mode de réalisation, la composition aqueuse stérilisée selon l'invention est caractérisée en ce que la teneur en polyol est de 35 mg/g en poids total de ladite composition.

Dans un mode de réalisation, la composition aqueuse stérilisée selon l'invention est caractérisée en ce que le polyol est le mannitol et sa teneur est comprise entre 10 et 40 mg/g en poids total de ladite composition.

Dans un mode de réalisation, la composition aqueuse stérilisée selon l'invention est caractérisée en ce que le polyol est le mannitol et sa teneur est comprise entre 15 et 30 mg/g en poids total de ladite composition.

Dans un mode de réalisation, la composition aqueuse stérilisée selon l'invention est caractérisée en ce que le polyol est le mannitol et sa teneur est comprise entre 15 et 25 mg/g en poids total de ladite composition.

Dans un mode de réalisation, la composition aqueuse stérilisée selon l'invention est caractérisée en ce que le polyol est le mannitol et sa teneur est comprise entre 20 et 40 mg/g en poids total de ladite composition.

Dans un mode de réalisation, la composition aqueuse stérilisée selon l'invention est caractérisée en ce que le polyol est le mannitol et sa teneur est comprise entre 25 et 35 mg/g en poids total de ladite composition.

Dans un mode de réalisation, la composition aqueuse stérilisée selon l'invention est caractérisée en ce que le polyol est le mannitol et sa teneur est de 35 mg/g en poids total de ladite composition.

Dans un mode de réalisation, la composition aqueuse stérilisée selon l'invention est caractérisée en ce que le polyol est le sorbitol et sa teneur est comprise entre 10 et 40 mg/g en poids total de ladite composition.

Dans un mode de réalisation, la composition aqueuse stérilisée selon l'invention est caractérisée en ce que le polyol est le sorbitol et sa teneur est comprise entre 15 et 30 mg/g en poids total de ladite composition.

Dans un mode de réalisation, la composition aqueuse stérilisée selon l'invention est caractérisée en ce que le polyol est le sorbitol et sa teneur est comprise entre 15 et 25 mg/g en poids total de ladite composition.

Dans un mode de réalisation, la composition aqueuse stérilisée selon l'invention est caractérisée en ce que le polyol est le sorbitol et sa teneur est comprise entre 20 et 40 mg/g en poids total de ladite composition.

Dans un mode de réalisation, la composition aqueuse stérilisée selon l'invention est caractérisée en ce que le polyol est le sorbitol et sa teneur est comprise entre 25 et 35 mg/g en poids total de ladite composition.

Dans un mode de réalisation, la composition aqueuse stérilisée selon l'invention est caractérisée en ce que le polyol est le sorbitol et sa teneur est de 35 mg/g en poids total de ladite composition.

Dans un mode de réalisation, la composition aqueuse stérilisée selon l'invention est caractérisée en ce que le polyol est le maltitol et sa teneur est comprise entre 10 et 40 mg/g en poids total de ladite composition.

Dans un mode de réalisation, la composition aqueuse stérilisée selon l'invention est caractérisée en ce que le polyol est le maltitol et sa teneur est comprise entre 15 et 30 mg/g en poids total de ladite composition.

Dans un mode de réalisation, la composition aqueuse stérilisée selon l'invention est caractérisée en ce que le polyol est le maltitol et sa teneur est comprise entre 15 et 25 mg/g en poids total de ladite composition.

Dans un mode de réalisation, la composition aqueuse stérilisée selon l'invention est caractérisée en ce que le polyol est le maltitol et sa teneur est comprise entre 20 et 40 mg/g en poids total de ladite composition.

Dans un mode de réalisation, la composition aqueuse stérilisée selon l'invention est caractérisée en ce que le polyol est le maltitol et sa teneur est comprise entre 25 et 35 mg/g en poids total de ladite composition.

Dans un mode de réalisation, la composition aqueuse stérilisée selon l'invention est caractérisée en ce que le polyol est le maltitol et sa teneur est de 35 mg/g en poids total de ladite composition.

Dans un mode de réalisation, la composition aqueuse stérilisée selon l'invention est caractérisée en ce que le polyol est le glycérol et sa teneur est comprise entre 10 et 40 mg/g en poids total de ladite composition.

Dans un mode de réalisation, la composition aqueuse stérilisée selon l'invention est caractérisée en ce que le polyol est le glycérol et sa teneur est comprise entre 15 et 30 mg/g en poids total de ladite composition.

Dans un mode de réalisation, la composition aqueuse stérilisée selon l'invention est caractérisée en ce que le polyol est le glycérol et sa teneur est comprise entre 15 et 25 mg/g en poids total de ladite composition.

Dans un mode de réalisation, la composition aqueuse stérilisée selon l'invention est caractérisée en ce que le polyol est le glycérol et sa teneur est comprise entre 20 et 40 mg/g en poids total de ladite composition.

Dans un mode de réalisation, la composition aqueuse stérilisée selon l'invention est caractérisée en ce que le polyol est le glycérol et sa teneur est comprise entre 25 et 35 mg/g en poids total de ladite composition.

Dans un mode de réalisation, la composition aqueuse stérilisée selon l'invention est caractérisée en ce que le polyol est le maltitol.

Dans un mode de réalisation, la composition aqueuse stérilisée selon l'invention est caractérisée en ce que le polyol est le glycérol et sa teneur est de 35 mg/g en poids total de ladite composition.

Dans un mode de réalisation, la composition aqueuse stérilisée selon l'invention est caractérisée en ce que la mépivacaïne est libérée librement *in vivo*.

Dans un mode de réalisation, la composition aqueuse stérilisée selon l'invention est caractérisée en ce que ladite composition comprend en outre au moins un composé additionnel.

Dans un mode de réalisation, la composition aqueuse stérilisée selon l'invention est caractérisée en ce que la teneur en composé additionnel est comprise entre 0,1 et 100 mg/g de poids total de ladite composition.

Dans un mode de réalisation, la composition aqueuse stérilisée selon l'invention est caractérisée en ce que la teneur en composé additionnel est comprise entre 1 et 50 mg/g de poids total de ladite composition.

Dans un mode de réalisation, la composition aqueuse stérilisée selon l'invention est caractérisée en ce que le composé additionnel est la diméthyl sulfone, ci-après DMS.

Dans un mode de réalisation, la composition aqueuse stérilisée selon l'invention est caractérisée en ce que le composé additionnel est un sel hydrosoluble de sucrose octasulfate, ci-après SOS.

Dans un mode de réalisation, la composition aqueuse stérilisée selon l'invention est caractérisée en ce que le composé additionnel est un dérivé de vitamine C.

Dans un mode de réalisation, le dérivé de vitamine C est un sel d'ascorbyl phosphate de magnésium, ci-après MAP.

Dans un mode de réalisation, la composition aqueuse stérilisée selon l'invention est caractérisée en ce que le composé additionnel appartient à la famille des catécholamines.

Dans un mode de réalisation, la composition aqueuse stérilisée selon l'invention est caractérisée en ce que le composé additionnel appartenant à la famille des catécholamines, est l'épinéphrine.

Dans un mode de réalisation, la composition aqueuse stérilisée selon l'invention est caractérisée en ce que la teneur en composé additionnel est comprise entre 0,01 et 10 % en poids par rapport au poids total de ladite composition.

Dans un mode de réalisation, la composition aqueuse stérilisée selon l'invention est caractérisée en ce que la teneur en composé additionnel est comprise entre 0,1 et 5 % en poids par rapport au poids total de ladite composition.

Dans un mode de réalisation, la teneur totale en composés additionnels est comprise entre 0,01 mg/g et 40 mg/g de poids total de ladite composition.

Dans un mode de réalisation, la teneur totale en composés additionnels est comprise entre 0,1 mg/g et 10 mg/g de poids total de ladite composition.

Dans un mode de réalisation, la teneur totale en composés additionnels est comprise entre 0,1 mg/g et 1 mg/g de poids total de ladite composition.

Dans un mode de réalisation, le composé additionnel est la diméthyl sulfone et sa teneur est comprise entre 1 et 10 mg/g en poids total de ladite composition.

Dans un mode de réalisation, le composé additionnel est un sel hydrosoluble de sucrose octasulfate et sa teneur est comprise entre 1 et 10 mg/g en poids total de ladite composition.

Dans un mode de réalisation, le composé additionnel est un sel d'ascorbyl phosphate de magnésium et sa teneur est comprise entre 0,3 et 10 mg/g en poids total de ladite composition.

L'invention concerne également un procédé de fabrication d'une composition aqueuse stérilisée selon l'invention.

Dans un mode de réalisation le procédé selon l'invention est caractérisé en ce qu'il comprend au moins :

- Une étape d'hydratation dans une solution tampon à pH proche du pH physiologique d'au moins un acide hyaluronique ou l'un de ses sels, seuls ou en mélange, pour obtenir un hydrogel,
- Une étape d'incorporation de la mépivacaïne en solution aqueuse avec l'hydrogel obtenu à l'étape précédente,
- Une étape d'homogénéisation, et
- Une étape de stérilisation.

Dans un mode de réalisation, l'acide hyaluronique est sous forme de fibres.

Dans un mode de réalisation, l'acide hyaluronique est sous forme de paillettes.

Dans un mode de réalisation, la solution tampon est une solution aqueuse de tampon phosphate.

Dans un mode de réalisation, le pH de la solution de mépivacaïne est ajusté à une valeur comprise entre 6,5 et 7 avant son introduction dans le gel et/ou hydogel.

Dans un mode de réalisation, la solution de mépivacaïne est incorporée dans le gel selon le procédé décrit dans la demande de brevet français 13/52971 au nom de la demanderesse.

Dans un mode de réalisation, le pH du gel et/ou hydrogel est ajusté à une valeur comprise entre 7,7 et 8 avant l'introduction de la solution de mépivacaïne dont le pH n'est pas ajusté.

Dans un mode de réalisation, la solution de mépivacaïne est incorporé dans le gel selon le procédé décrit dans la demande de brevet WO 2010/015901 au nom d'Allergan.

Dans un mode de réalisation, le procédé selon l'invention est caractérisé en ce que l'étape d'hydratation est réalisée à température ambiante.

Dans un mode de réalisation, le procédé selon l'invention est caractérisé en ce que l'étape d'homogénéisation est réalisée à température ambiante.

Dans un mode de réalisation, le procédé selon l'invention est caractérisé en ce qu'il comprend en outre au moins une étape de conditionnement du mélange homogénéisé dans des seringues.

Dans un mode de réalisation, le procédé selon l'invention est caractérisé en ce qu'il comprend en outre au moins une étape de conditionnement du mélange homogénéisé dans des flacons uni-doses.

Dans un mode de réalisation, le procédé est caractérisé en ce qu'il comprend au moins une étape de stérilisation.

Dans un mode de réalisation, ladite étape de stérilisation est effectuée après l'étape de conditionnement.

Dans un mode de réalisation, ladite étape de stérilisation est effectuée par la chaleur, la chaleur humide, le rayonnement gamma($\gamma$), ou par faisceau d'électrons accélérés (Electron-beam).

Dans un mode de réalisation, l'étape de stérilisation est réalisée après le conditionnement par autoclavage à la vapeur.

Dans un mode de réalisation, l'étape de stérilisation est réalisée après le conditionnement par irradiation par rayonnement gamma (y) ou par faisceau d'électrons accélérés (Electron-beam).

Dans un mode de réalisation, le procédé selon l'invention est caractérisé en ce que la stérilisation par autoclavage à la vapeur est réalisée après le conditionnement à une température de 121 à 134°C, pendant une durée adaptée à la température.

Par exemple la stérilisation par autoclavage à la vapeur est réalisée à une température comprise entre 127 et 130°C pendant une durée comprise entre 1 et 20 min.

Dans un mode de réalisation, le procédé selon l'invention est caractérisé en ce qu'il comprend en outre au moins une étape de réticulation.

Dans un mode de réalisation, le procédé selon l'invention est caractérisé en ce que l'étape de réticulation se situe entre l'étape d'hydratation et l'étape d'incorporation de la mépivacaïne.

Dans un mode de réalisation, le procédé selon l'invention est caractérisé en ce que l'étape de réticulation est réalisée au moyen d'au moins un agent de réticulation.

Dans un mode de réalisation, le procédé selon l'invention est caractérisé en ce que l'agent de réticulation est bi- ou polyfonctionnel.

Dans un mode de réalisation, le procédé selon l'invention est caractérisé en ce que l'agent de réticulation bi- ou polyfonctionnel est choisi parmi le groupe constitué par l'éther d'éthylèneglycoldiglycidyle, l'éther de butanedioldiglycidyle (BDDE), l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde, l'épichlorohydrine ou bien encore le glutaraldéhyde, les carbodiimides tels que par exemple le 1-éthyl-3-[3-dimethylaminopropyl]carbodiimide hydrochloride (EDC).

Dans un mode de réalisation, le procédé selon l'invention est caractérisé en ce que l'agent de réticulation bifonctionnel est l'éther de butanedioldiglycidyle (BDDE) ou le 1,2,7,8-diépoxyoctane.

Dans un mode de réalisation, le procédé de fabrication selon l'invention est caractérisé en ce que l'étape de réticulation est mise en œuvre selon les techniques connues de l'homme du métier.

Dans un mode de réalisation, le procédé selon l'invention est caractérisé en ce qu'il comprend après l'étape de réticulation, au moins une étape de purification et lavage mise en œuvre selon les techniques connues de l'homme du métier.

Dans un mode de réalisation, le procédé selon l'invention est caractérisé en ce qu'il comprend en outre au moins une étape d'incorporation d'au moins un antioxydant.

Dans un mode de réalisation, le au moins antioxydant est choisi parmi les polyols.

Dans un mode de réalisation, les polyols sont choisis dans le groupe constitué par le glycérol, le sorbitol, le propylène glycol, le xylitol, le mannitol, l'érythritol, le maltitol et le lactitol, seul ou en mélange.

Dans un mode de réalisation, le procédé selon l'invention est caractérisé en ce qu'il comprend en outre au moins une étape de mélange d'une solution d'au moins un composé additionnel avec l'hydrogel obtenu à l'étape d'hydratation.

Dans un mode de réalisation, le procédé selon l'invention est caractérisé en ce que l'étape de mélange d'une solution d'au moins un composé additionnel avec l'hydrogel obtenu à l'étape d'hydratation se situe avant l'étape d'homogénéisation.

Dans un mode de réalisation, le procédé selon l'invention est caractérisé en ce que l'étape de mélange d'une solution d'au moins un composé additionnel avec l'hydrogel obtenu à l'étape d'hydratation est réalisée à une température adaptée au procédé de fabrication. Dans un mode de réalisation elle est réalisée à température ambiante.

L'invention concerne également un procédé d'obtention d'une composition aqueuse stérilisée d'acide hyaluronique comprenant un anesthésique local, ladite composition ayant des propriétés rhéologiques après stérilisation à la chaleur supérieures aux propriétés rhéologiques d'une composition comprenant de la lidocaïne, caractérisé en ce que la lidocaïne est substituée par une quantité équivalente, à un même pH, de mépivacaïne.

On entend par quantité équivalente soit une quantité équivalente en masse, en moles ou de biodisponibilité équivalente à un pH proche du pH physiologique.

Dans ledit procédé, la composition obtenue est définie comme la composition selon l'invention.

L'invention concerne également l'utilisation de la mépivacaïne en substitution de la lidocaïne en quantité équivalente pour obtenir une composition d'acide hyaluronique comprenant un anesthésique local dont les propriétés rhéologiques après stérilisation à la chaleur sont supérieures aux propriétés rhéologiques d'une même composition d'acide hyaluronique comprenant de la lidocaïne.

Dans ladite utilisation, la composition obtenue est définie comme la composition selon l'invention.

On entend par quantité équivalente soit une quantité équivalente en masse, en moles ou de biodisponibilité équivalente.

On entend par acide hyaluronique, l'acide hyaluronique, réticulé ou non réticulé, seul ou en mélange, éventuellement modifié chimiquement par substitution, seul ou en mélange, éventuellement sous forme de l'un de ses sels, seul ou en mélange.

On entend par anesthésique local, un anesthésique local ou l'un de ses sels, seuls ou en mélange.

On entend par mépivacaïne, la mépivacaïne ou l'un de ses sels, seuls ou en mélange.

On entend par lidocaïne, la lidocaïne ou l'un de ses sels, seuls ou en mélange.

On entend par propriétés rhéologiques, le module élastique (G') et ou la viscosité ($\eta$).

On entend par propriétés rhéologiques supérieures, que les valeurs du module élastique et ou de la viscosité sont plus élevées.

On entend par « en substitution » la formulation de gels au sein desquels est incorporée de la mépivacaïne en lieu et place de la lidocaïne.

L'invention concerne également, l'utilisation de la mépivacaïne pour améliorer la résistance à la dégradation des propriétés rhéologiques d'une composition aqueuse stérilisée injectable d'acide hyaluronique lors de la stérilisation à la chaleur.

L'invention concerne également, l'utilisation de la mépivacaine pour améliorer la résistance à la dégradation des propriétés rhéologiques d'une composition aqueuse d'acide hyaluronique comprenant un anesthésique local lors de la stérilisation à la chaleur.

L'invention concerne également, l'utilisation de la mépivacaïne dans une composition aqueuse d'acide hyaluronique, ladite composition présentant une dégradation des propriétés rhéologiques lors de la stérilisation à la chaleur inférieure à celle d'une même composition d'acide hyaluronique comprenant un autre anesthésique local.

L'invention concerne également, un procédé d'amélioration de la résistance à la dégradation des propriétés rhéologiques d'une composition aqueuse injectable d'acide hyaluronique lors de la stérilisation à la chaleur, caractérisé en ce que ladite composition comprend de la mépivacaïne.

L'invention concerne également, un procédé d'amélioration de la résistance à la dégradation des propriétés rhéologiques d'une composition aqueuse injectable d'acide hyaluronique comprenant un anesthésique local lors de la stérilisation à la chaleur, caractérisé en ce que ladite composition comprend de la mépivacaïne.

L'invention concerne également, un procédé d'amélioration de la résistance à la dégradation des propriétés rhéologiques lors de la stérilisation à la chaleur d'une composition aqueuse injectable d'acide hyaluronique comprant un anesthésique local, par substitution de la lidocaïne par une quantité équivalente de mépivacaïne.

Dans un mode de réalisation, les utilisations ou les procédés sont caractérisés en ce que la mépivacaïne est utilisée en quantité équivalente.

Dans un mode de réalisation, les utilisations ou les procédés sont caractérisés en ce que la stérilisation est réalisée par autoclavage à la vapeur.

Dans un mode de réalisation, les utilisations ou les procédés sont caractérisés en ce que l'autre anesthésique local est choisi dans le groupe constitué par la lidocaïne et la prilocaïne.

Dans un mode de réalisation, les utilisations ou les procédés sont caractérisés en ce que l'autre anesthésique local est la lidocaïne.

Dans un mode de réalisation, les utilisations ou les procédés sont caractérisés en ce que la composition comprend en outre un ou plusieurs polyol(s).

Dans un mode de réalisation, les utilisations ou les procédés sont caractérisés en ce que le(s) un ou plusieurs polyol(s) est (sont) choisi(s) dans le groupe constitué par le glycérol, le sorbitol, le propylène glycol, le xylitol, le mannitol, l'érythritol, le maltitol et le lactitol, seul ou en mélange.

Dans un mode de réalisation, les utilisations ou les procédés sont caractérisés en ce que le(s) un ou plusieurs polyol(s) est (sont) choisi(s) dans le groupe constitué par le mannitol, le sorbitol, le maltitol, et le glycérol, seul ou en mélange.

Dans un mode de réalisation, les utilisations ou les procédés sont caractérisés en ce que le(s) un ou plusieurs polyol(s) est (sont) choisi(s) dans le groupe constitué par le mannitol et le sorbitol, seul ou en mélange.

Dans un mode de réalisation, les utilisations ou les procédés sont caractérisés en ce qu'au moins un polyol est le mannitol.

Dans un mode de réalisation, les utilisations ou les procédés sont caractérisés en ce que le polyol est le mannitol.

Dans un mode de réalisation, les utilisations ou les procédés sont caractérisés en ce qu'au moins un polyol est le sorbitol.

Dans un mode de réalisation, les utilisations ou les procédés sont caractérisés en ce que le polyol est le sorbitol.

Dans un mode de réalisation, les utilisations ou les procédés sont caractérisés en ce qu'au moins un polyol est le maltitol.

Dans un mode de réalisation, les utilisations ou les procédés sont caractérisés en ce que le polyol est le maltitol.

Dans un mode de réalisation, les utilisations ou les procédés sont caractérisés en ce qu'au moins un polyol est le glycérol.

Dans un mode de réalisation, les utilisations ou les procédés sont caractérisés en ce que le polyol est le glycérol.

Dans un mode de réalisation, les utilisations ou les procédés sont caractérisés en ce que le ou les polyol(s) a(ont) une teneur comprise entre 10 et 40 mg/g en poids total de ladite composition.

Dans un mode de réalisation, les utilisations ou les procédés sont caractérisés en ce que le ou les polyol(s) a(ont) une teneur comprise entre 15 et 30 mg/g en poids total de ladite composition.

Dans un mode de réalisation, les utilisations ou les procédés sont caractérisés en ce que le ou les polyol(s) a(ont) une teneur comprise entre 15 et 25 mg/g en poids total de ladite composition.

Dans un mode de réalisation, les utilisations ou les procédés sont caractérisés en ce que le ou les polyol(s) a(ont) une teneur comprise entre 20 et 40 mg/g en poids total de ladite composition.

Dans un mode de réalisation, les utilisations ou les procédés sont caractérisés en ce que le ou les polyol(s) a(ont) une teneur comprise entre 25 et 35 mg/g en poids total de ladite composition.

Dans un mode de réalisation, les utilisations ou les procédés sont caractérisés en ce que le ou les polyol(s) a(ont) une teneur de 35 mg/g en poids total de ladite composition.

Dans un mode de réalisation, les utilisations ou les procédés sont caractérisés en ce que le polyol est le mannitol et sa teneur est comprise entre 10 et 40 mg/g en poids total de ladite composition.

Dans un mode de réalisation, les utilisations ou les procédés sont caractérisés en ce que le polyol est le mannitol et sa teneur est comprise entre 15 et 30 mg/g en poids total de ladite composition.

Dans un mode de réalisation, les utilisations ou les procédés sont caractérisés en ce que le polyol est le mannitol et sa teneur est comprise entre 15 et 25 mg/g en poids total de ladite composition.

Dans un mode de réalisation, les utilisations ou les procédés sont caractérisés en ce que le polyol est le mannitol et sa teneur est comprise entre 20 et 40 mg/g en poids total de ladite composition.

Dans un mode de réalisation, les utilisations ou les procédés sont caractérisés en ce que le polyol est le mannitol et sa teneur est comprise entre 25 et 35 mg/g en poids total de ladite composition.

Dans un mode de réalisation, les utilisations ou les procédés sont caractérisés en ce que le polyol est le mannitol et sa teneur est de 35 mg/g en poids total de ladite composition.

Dans un mode de réalisation, les utilisations ou les procédés sont caractérisés en ce que le polyol est le sorbitol et sa teneur est comprise entre 10 et 40 mg/g en poids total de ladite composition.

Dans un mode de réalisation, les utilisations ou les procédés sont caractérisés en ce que le polyol est le sorbitol et sa teneur est comprise entre 15 et 30 mg/g en poids total de ladite composition.

Dans un mode de réalisation, les utilisations ou les procédés sont caractérisés en ce que le polyol est le sorbitol et sa teneur est comprise entre 15 et 25 mg/g en poids total de ladite composition.

Dans un mode de réalisation, les utilisations ou les procédés sont caractérisés en ce que le polyol est le sorbitol et sa teneur est comprise entre 20 et 40 mg/g en poids total de ladite composition.

Dans un mode de réalisation, les utilisations ou les procédés sont caractérisés en ce que le polyol est le sorbitol et sa teneur est comprise entre 25 et 35 mg/g en poids total de ladite composition.

Dans un mode de réalisation, les utilisations ou les procédés sont caractérisés en ce que le polyol est le sorbitol et sa teneur est de 35 mg/g en poids total de ladite composition.

Dans un mode de réalisation, les utilisations ou les procédés sont caractérisés en ce que le polyol est le maltitol et sa teneur est comprise entre 10 et 40 mg/g en poids total de ladite composition.

Dans un mode de réalisation, les utilisations ou les procédés sont caractérisés en ce que le polyol est le maltitol et sa teneur est comprise entre 15 et 30 mg/g en poids total de ladite composition.

Dans un mode de réalisation, les utilisations ou les procédés sont caractérisés en ce que le polyol est le maltitol et sa teneur est comprise entre 10 et 25mg/g en poids total de ladite composition.

Dans un mode de réalisation, les utilisations ou les procédés sont caractérisés en ce que le polyol est le maltitol et sa teneur est comprise entre 20 et 40 mg/g en poids total de ladite composition.

Dans un mode de réalisation, les utilisations ou les procédés sont caractérisés en ce que le polyol est le maltitol et sa teneur est comprise entre 25 et 35 mg/g en poids total de ladite composition.

Dans un mode de réalisation, les utilisations ou les procédés sont caractérisés en ce que le polyol est le maltitol et sa teneur est de 35 mg/g en poids total de ladite composition.

Dans un mode de réalisation, les utilisations ou les procédés sont caractérisés en ce que le polyol est le glycérol et sa teneur est comprise entre 10 et 40 mg/g en poids total de ladite composition.

Dans un mode de réalisation, les utilisations ou les procédés sont caractérisés en ce que le polyol est le glycérol et sa teneur est comprise entre 15 et 30 mg/g en poids total de ladite composition.

Dans un mode de réalisation, les utilisations ou les procédés sont caractérisés en ce que le polyol est le glycérol et sa teneur est comprise entre 10 et 25 mg/g en poids total de ladite composition.

Dans un mode de réalisation, les utilisations ou les procédés sont caractérisés en ce que le polyol est le glycérol et sa teneur est comprise entre 20 et 40 mg/g en poids total de ladite composition.

Dans un mode de réalisation, les utilisations ou les procédés sont caractérisés en ce que le polyol est le glycérol et sa teneur est comprise entre 25 et 35 mg/g en poids total de ladite composition.

Dans un mode de réalisation, les utilisations ou les procédés sont caractérisés en ce que le polyol est le glycérol et sa teneur est de 35 mg/g en poids total de ladite composition.

Dans un mode de réalisation, les utilisations ou les procédés sont caractérisés en ce que l'étape de stérilisation est réalisée par autoclavage à la vapeur à une température comprise entre 121 à 134°C, pendant une durée adaptée à la température.

Par exemple la stérilisation par autoclavage à la vapeur est réalisée à une température comprise entre 127 et 130°C pendant une durée comprise entre 1 et 20 min.

Dans un mode de réalisation, les utilisations ou les procédés sont caractérisés en ce que la composition comprend en outre un antioxydant.

Dans un mode de réalisation, les utilisations ou les procédés sont caractérisés en ce que le ratio massique entre la concentration en acide hyaluronique [HA] et la concentration en mépivacaïne [MEPI] : [HA]/[MEPI] est supérieur ou égal à 0,1 ; [HA]/[MEPI] $\geq$ 0,1.

Dans un mode de réalisation, les utilisations ou les procédés sont caractérisés en ce que le ratio massique entre la concentration en acide hyaluronique [HA] et la concentration en mépivacaïne [MEPI] : [HA]/[MEPI] est compris entre 0,1 et 50, $0,1 \leq$ [HA]/[MEPI] $\leq$ 50.

Dans un mode de réalisation, les utilisations ou les procédés sont caractérisés en ce que le ratio massique entre la concentration en acide hyaluronique [HA] et la concentration en mépivacaïne [MEPI] : [HA]/[MEPI] est compris entre 0,5 et 40, 0,5 ≤ [HA]/[MEPI] ≤ 40.

Dans un mode de réalisation, les utilisations ou les procédés sont caractérisés en ce que le ratio massique entre la concentration en acide hyaluronique [HA] et la concentration en mépivacaïne [MEPI] : [HA]/[MEPI] est compris entre 1 et 30, 1 ≤ [HA]/[MEPI] ≤ 30.

Dans un mode de réalisation, les utilisations ou les procédés sont caractérisés en ce que le ratio massique entre la concentration en acide hyaluronique [HA] et la concentration en mépivacaïne [MEPI] : [HA]/[MEPI] est compris entre 2 et 20, 2 ≤ [HA]/[MEPI] ≤ 20.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est compris entre 7/3 et 26/3, 7/3 ≤ [HA]/[MEPI] ≤ 26/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est compris entre 2 et 20/3, 2 ≤ [HA]/[MEPI] ≤ 20/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est compris entre 2 et 10/3, 2 ≤ [HA]/[MEPI] ≤ 10/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 20.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 26/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 20/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 10/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 7/3.

Dans un mode de réalisation, les utilisations ou les procédés sont caractérisés en ce que le ratio massique entre la concentration en acide hyaluronique [HA] et la concentration en mépivacaïne [MEPI], [HA]/[MEPI] est de 2.

Dans un mode de réalisation, les utilisations ou les procédés sont caractérisés en ce que la concentration en mépivacaïne [MEPI] est comprise entre 0,01 mg/g et 50 mg/g de poids total de ladite composition.

Dans un mode de réalisation, les utilisations ou les procédés sont caractérisés en ce que la concentration en mépivacaïne [MEPI] est comprise entre 0,01 mg/g et 50 mg/g de poids total de ladite composition.

Dans un mode de réalisation, les utilisations ou les procédés sont caractérisés en ce que la concentration en mépivacaïne [MEPI] est comprise entre 0,05 mg/g et 45 mg/g de poids total de ladite composition.

Dans un mode de réalisation, les utilisations ou les procédés sont caractérisés en ce que la concentration en mépivacaïne [MEPI] est comprise entre 0,1 mg/g et 40 mg/g de poids total de ladite composition.

Dans un mode de réalisation, les utilisations ou les procédés sont caractérisés en ce que la concentration en mépivacaïne [MEPI] est comprise entre 0,2 mg/g et 30 mg/g de poids total de ladite composition.

Dans un mode de réalisation, les utilisations ou les procédés sont caractérisés en ce que la concentration en mépivacaïne [MEPI] est comprise entre 0,5 mg/g et 20 mg/g de poids total de ladite composition.

Dans un mode de réalisation, les utilisations ou les procédés sont caractérisés en ce que la concentration en mépivacaïne [MEPI] est comprise entre 1 mg/g et 15 mg/g de poids total de ladite composition.

Dans un mode de réalisation, les utilisations ou les procédés sont caractérisés en ce que la concentration en mépivacaïne [MEPI] est comprise entre 1 mg/g et 10 mg/g de poids total de ladite composition.

Dans un mode de réalisation, les utilisations ou les procédés sont caractérisés en ce que la concentration en mépivacaïne [MEPI] est comprise entre 1 mg/g et 6 mg/g de poids total de ladite composition.

Dans un mode de réalisation, les utilisations ou les procédés sont caractérisés en ce que la concentration en mépivacaïne [MEPI] est comprise entre 1 mg/g et 5 mg/g de poids total de ladite composition.

Dans un mode de réalisation, les utilisations ou les procédés sont caractérisés en ce que la concentration en mépivacaïne [MEPI] est comprise entre 2 mg/g et 5 mg/g de poids total de ladite composition.

Dans un mode de réalisation, les utilisations ou les procédés sont caractérisés en ce que la concentration en mépivacaïne [MEPI] est comprise entre 6 mg/g et 10 mg/g de poids total de ladite composition.

Dans un mode de réalisation, les utilisations ou les procédés sont caractérisés en ce que la concentration en mépivacaïne [MEPI] est de 1 mg/g de poids total de ladite composition.

Dans un mode de réalisation, les utilisations ou les procédés sont caractérisés en ce que la concentration en mépivacaïne [MEPI] est de 3 mg/g de poids total de ladite composition.

Dans un mode de réalisation, les utilisations ou les procédés sont caractérisés en ce que la concentration en mépivacaïne [MEPI] est de 4 mg/g de poids total de ladite composition.

Dans un mode de réalisation, les utilisations ou les procédés sont caractérisés en ce que la concentration en mépivacaïne [MEPI] est de 5 mg/g de poids total de ladite composition.

Dans un mode de réalisation, les utilisations ou les procédés sont caractérisés en ce que la concentration en mépivacaïne [MEPI] est de 6 mg/g de poids total de ladite composition.

Dans un mode de réalisation, les utilisations ou les procédés sont caractérisés en ce que la concentration en mépivacaïne [MEPI] est de 10 mg/g de poids total de ladite composition.

Dans un mode de réalisation, les utilisations ou les procédés sont caractérisés en ce que la mépivacaïne est choisie dans le groupe comprenant la mépivacaïne ou l'un de ses sels pharmaceutiquement acceptables.

Dans un mode de réalisation, les utilisations ou les procédés sont caractérisés en ce que la mépivacaïne est choisie dans le groupe constitué par l'hydrochlorure de mépivacaïne racémique, la mépivacaïne racémique, l'hydrochlorure de (R)-mépivacaïne, l'hydrochlorure de (S)-mépivacaïne, la (R)-mépivacaïne et la (S)-mépivacaïne ou l'un de leurs sels pharmaceutiquement acceptables.

Dans un mode de réalisation, les utilisations ou les procédés sont caractérisés en ce que la mépivacaïne est l'hydrochlorure de mépivacaïne racémique.

Dans un mode de réalisation, les utilisations ou les procédés sont caractérisés en ce que la mépivacaïne est l'hydrochlorure de (R)-mépivacaïne.

Dans un mode de réalisation, les utilisations ou les procédés sont caractérisés en ce que la mépivacaïne est l'hydrochlorure de (S)-mépivacaïne.

Dans un mode de réalisation, les utilisations ou les procédés sont caractérisés en ce que la mépivacaïne est la mépivacaïne racémique.

Dans un mode de réalisation, les utilisations ou les procédés sont caractérisés en ce que la mépivacaïne est la (R)-mépivacaïne.

Dans un mode de réalisation, les utilisations ou les procédés sont caractérisés en ce que la mépivacaïne est la (S)-mépivacaïne.

Dans un mode de réalisation, les utilisations ou les procédés sont caractérisés en ce que la concentration en acide hyaluronique [HA] est comprise entre 2 mg/g et 50 mg/g de poids total de ladite composition.

Dans un mode de réalisation, les utilisations ou les procédés sont caractérisés en ce que la concentration en acide hyaluronique [HA] est comprise entre 4 mg/g et 40 mg/g de poids total de ladite composition.

Dans un mode de réalisation, les utilisations ou les procédés sont caractérisés en ce que la concentration en acide hyaluronique [HA] est comprise entre 5 mg/g et 30 mg/g de poids total de ladite composition.

Dans un mode de réalisation, les utilisations ou les procédés sont caractérisés en ce que la concentration en acide hyaluronique [HA] est comprise entre 10 mg/g et 30 mg/g de poids total de ladite composition.

Dans un mode de réalisation, les utilisations ou les procédés sont caractérisés en ce que la concentration en acide hyaluronique [HA] est de 20 mg/g de poids total de ladite composition.

Dans un mode de réalisation, les utilisations ou les procédés sont caractérisés en ce que la teneur totale en acide hyaluronique est comprise entre 0,2 et 5% en poids par rapport au poids total de ladite composition.

Dans un mode de réalisation, les utilisations ou les procédés sont caractérisés en ce que la teneur totale en acide hyaluronique est supérieure ou égale à 1 % en poids par rapport au poids total de ladite composition.

Dans un mode de réalisation, les utilisations ou les procédés sont caractérisés en ce que la composition aqueuse stérilisée comprend au moins un acide hyaluronique non réticulé ou l'un de ses sels, seul ou en mélange.

Dans un mode de réalisation, les utilisations ou les procédés sont caractérisés en ce que la composition aqueuse stérilisée comprend au moins un acide hyaluronique réticulé ou l'un de ses sels, seul ou en mélange.

Dans un mode de réalisation, les utilisations ou les procédés sont caractérisés en ce que la composition aqueuse stérilisée comprend au moins un acide hyaluronique co-réticulé ou l'un de ses sels, seul ou en mélange.

Dans un mode de réalisation, les utilisations ou les procédés sont caractérisés en ce que la composition aqueuse stérilisée comprend au moins un acide hyaluronique modifié chimiquement par substitution et réticulé, ou l'un de ses sels, seul ou en mélange.

Dans un mode de réalisation, les utilisations ou les procédés sont caractérisés en ce que l'acide hyaluronique est doublement réticulé tel que décrit dans la demande de brevet WO2000/046253 au nom de Fermentech Medical Limited.

Dans un mode de réalisation, les utilisations ou les procédés sont caractérisés en ce que la composition aqueuse stérilisée comprend un mélange d'acides hyaluroniques, ou l'un de leurs sels, réticulés.

Dans un mode de réalisation, les utilisations ou les procédés sont caractérisés en ce que le mélange d'acides hyaluroniques, ou l'un de leurs sels, réticulés est un mélange monophasique tel que celui décrit dans la demande de brevet WO2009/071697 au nom de la demanderesse.

Dans un mode de réalisation, les utilisations ou les procédés sont caractérisés en ce que le mélange d'acides hyaluroniques, ou l'un de leurs sels, réticulés est un mélange obtenu par mélange de plusieurs acides hyaluroniques, ou l'un de leurs sels, de masses moléculaires différentes préalablement à leur réticulation, tel que décrit dans la demande de brevet WO2004/092222 au nom de Cornéal Industrie.

Dans un mode de réalisation, les utilisations ou les procédés sont caractérisés en ce que la composition aqueuse stérilisée comprend au moins un acide hyaluronique, ou l'un de ses sels, substitué par un groupement apportant des propriétés lipophile ou hydratante, comme par exemple les acides hyaluroniques substitués tels que décrits dans la demande de brevet FR 2 983 483 au nom de la demanderesse.

Dans un mode de réalisation, les utilisations ou les procédés sont caractérisés en ce que l'acide hyaluronique est sous forme de sel de sodium ou de potassium.

Dans un mode de réalisation, les utilisations ou les procédés sont caractérisés en ce que la masse moléculaire Mw de l'au moins un acide hyaluronique est comprise dans un intervalle de 0,01 MDa et 5 MDa.

Dans un mode de réalisation, les utilisations ou les procédés sont caractérisés en ce que la masse moléculaire Mw de l'au moins un acide hyaluronique est comprise dans un intervalle de 0,1 MDa et 3,5 MDa.

Dans un mode de réalisation, les utilisations ou les procédés sont caractérisés en ce que la masse moléculaire Mw de l'au moins un acide hyaluronique est comprise dans un intervalle de 1 MDa et 3 MDa.

Dans un mode de réalisation les utilisations ou les procédés sont caractérisés en ce que la masse moléculaire Mw de l'au moins un acide hyaluronique est de 1 MDa.

Dans un mode de réalisation les utilisations ou les procédés sont caractérisés en ce que la masse moléculaire Mw de l'au moins un acide hyaluronique est de 3 MDa.

Dans un mode de réalisation, les utilisations ou les procédés sont caractérisés en ce que l'acide hyaluronique réticulé présente un taux de réticulation X compris entre 0,001 et 0,5.

Dans un mode de réalisation, les utilisations ou les procédés sont caractérisés en ce que l'acide hyaluronique réticulé présente un taux de réticulation X compris entre 0,01 et 0,4.

Dans un mode de réalisation, les utilisations ou les procédés sont caractérisés en ce que l'acide hyaluronique réticulé présente un taux de réticulation X compris entre 0,1 et 0,3.

Dans un mode de réalisation, les utilisations ou les procédés sont caractérisés en ce que l'acide hyaluronique réticulé présente un taux de réticulation X de 0,08.

Dans un mode de réalisation, les utilisations ou les procédés sont caractérisés en ce que l'acide hyaluronique réticulé présente un taux de réticulation X de 0,06.

Dans un mode de réalisation, les utilisations ou les procédés sont caractérisés en ce que l'acide hyaluronique réticulé présente un taux de réticulation X de 0,12.

Dans un mode de réalisation, la composition aqueuse stérilisée selon l'invention comprend, en outre, un autre polysaccharide.

Dans un mode de réalisation, cet autre polysaccharide est choisi dans le groupe constitué par la cellulose et ses dérivés et/ou l'acide alginique ou l'un de leurs sels.

Dans un mode de réalisation, les utilisations ou les procédés sont caractérisés en ce que la composition aqueuse stérilisée comprend, en outre, un autre polysaccharide.

Dans un mode de réalisation, les utilisations ou les procédés sont caractérisés en ce que la composition aqueuse stérilisée selon l'invention comprend, en outre, un autre polysaccharide choisi dans le groupe constitué par la cellulose et ses dérivés et/ou l'acide alginique ou l'un de leurs sels.

Dans un mode de réalisation, les utilisations ou les procédés sont caractérisés en ce que la composition aqueuse stérilisée comprend en outre au moins un composé additionnel.

Dans un mode de réalisation, les utilisations ou les procédés sont caractérisés en ce que la teneur en composé additionnel dans la composition est comprise entre 0,1 et 100 mg/g de poids total de ladite composition.

Dans un mode de réalisation, les utilisations ou les procédés sont caractérisés en ce que la teneur en composé additionnel dans la composition est comprise entre 1 et 50 mg/g de poids total de ladite composition.

Dans un mode de réalisation, les utilisations ou les procédés sont caractérisés en ce que le composé additionnel est la diméthyl sulfone, ci-après DMS.

Dans un mode de réalisation, les utilisations ou les procédés sont caractérisés en ce que le composé additionnel est un sel hydrosoluble de sucrose octasulfate, ci-après SOS.

Dans un mode de réalisation, les utilisations ou les procédés sont caractérisés en ce que le composé additionnel est un dérivé de vitamine C.

Dans un mode de réalisation, les utilisations ou les procédés sont caractérisés en ce que le dérivé de vitamine C est un sel d'ascorbyl phosphate de magnésium, ci-après MAP.

Dans un mode de réalisation, les utilisations ou les procédés sont caractérisés en ce que le composé additionnel appartient à la famille des catécholamines.

Dans un mode de réalisation, les utilisations ou les procédés sont caractérisés en ce que le composé additionnel appartenant à la famille des catécholamines, est l'épinéphrine.

Dans un mode de réalisation, les utilisations ou les procédés sont caractérisés en ce que la teneur en composé additionnel est comprise entre 0,01 et 10 % en poids par rapport au poids total de ladite composition.

Dans un mode de réalisation, les utilisations ou les procédés sont caractérisés en ce que la teneur en composé additionnel est comprise entre 0,1 et 5 % en poids par rapport au poids total de ladite composition.

Dans un mode de réalisation, les utilisations ou les procédés sont caractérisés en ce que la teneur totale en composés additionnels est comprise entre 0,01 mg/g et 40 mg/g de poids total de ladite composition.

Dans un mode de réalisation, les utilisations ou les procédés sont caractérisés en ce que la teneur totale en composés additionnels est comprise entre 0,1 mg/g et 10 mg/g de poids total de ladite composition.

Dans un mode de réalisation, les utilisations ou les procédés sont caractérisés en ce que la teneur totale en composés additionnels est comprise entre 0,1 mg/g et 1 mg/g de poids total de ladite composition.

Dans un mode de réalisation, les utilisations ou les procédés sont caractérisés en ce que le composé additionnel est la diméthyl sulfone et sa teneur est comprise entre 1 et 10 mg/g en poids total de ladite composition.

Dans un mode de réalisation, les utilisations ou les procédés sont caractérisés en ce que le composé additionnel est un sel hydrosoluble de sucrose octasulfate et sa teneur est comprise entre 1 et 10 mg/g en poids total de ladite composition.

Dans un mode de réalisation, les utilisations ou les procédés sont caractérisés en ce que le composé additionnel est un sel d'ascorbyl phosphate de magnésium et sa teneur est comprise entre 0,3 et 10 mg/g en poids total de ladite composition.

L'invention concerne également l'utilisation d'une composition aqueuse stérilisée pour la formulation d'une composition de comblement de rides, de correction de défauts cutanés ou volumatrice (pommettes, menton, lèvres).

L'invention concerne également une composition aqueuse stérilisée selon l'invention, pour son utilisation en comblement de rides et/ou en correction de défauts cutanés.

L'invention concerne également l'utilisation d'une composition aqueuse stérilisée pour la formulation d'une composition injectable dans une articulation en remplacement ou en complément de liquide synovial déficient.

L'invention concerne également une composition aqueuse stérilisée selon l'invention, pour son utilisation en remplacement ou en complément de liquide synovial déficient.

L'invention concerne également l'utilisation d'une composition aqueuse stérilisée selon l'invention, pour la formulation d'une composition pour le comblement de rides.

L'invention concerne également l'utilisation d'une composition aqueuse stérilisée selon l'invention, pour la formulation d'une composition de viscosupplémentation.

L'invention concerne également une composition aqueuse stérilisée selon l'invention, pour son utilisation en tant que médicament.

Les applications visées sont plus particulièrement les applications communément répandues dans le cadre des viscoélastiques injectables et des polysaccharides utilisés ou potentiellement utilisables dans les pathologies ou traitements suivants :

- Injections esthétiques au niveau du visage : de comblement de rides, défauts cutanés ou volumatrices (pommettes, menton, lèvres) ;
- Injections volumatrices au niveau du corps : augmentation des seins et des fesses, augmentation du point G, vaginoplastie, reconstruction des lèvres vaginales, augmentation de la taille du pénis ;
- Traitement de l'arthrose, injection dans l'articulation en remplacement ou en complément du liquide synovial déficient ;
- Injection péri-urétrale pour le traitement de l'incontinence urinaire par insuffisance sphinctérienne ;
- Injection post-chirurgicale pour éviter les adhésions péritonéales notamment ;
- Injection suite à une chirurgie de la presbytie par incisions sclérales au laser ;
- Injection dans la cavité vitréenne ;
- Injection au cours de la chirurgie de la cataracte ;
- Injection dans les parties génitales.

Plus particulièrement, en chirurgie esthétique, en fonction de ses propriétés viscoélastiques et de rémanence, la composition aqueuse stérilisée obtenue selon le procédé de l'invention pourra être utilisée :

- pour le comblement des rides fines, moyennes ou profondes, et être injectée avec des aiguilles de diamètre fin (27 Gauge par exemple) ;
- comme volumateur avec une injection par des aiguilles de diamètre plus important, de 22 à 26 Gauge par exemple, et plus longues (30 à 40 mm par exemple); dans ce cas, son caractère cohésif permettra de garantir son maintien à l'emplacement de l'injection.

La composition aqueuse stérilisée selon l'invention trouve également une application importante en chirurgie articulaire et en chirurgie dentaire pour le comblement des poches parodontales par exemple.

Ces exemples d'utilisation ne sont nullement limitatifs, la composition aqueuse stérilisée selon la présente invention étant plus largement prévue pour :

- combler des volumes ;
- générer des espaces au sein de certains tissus, favorisant ainsi leur fonctionnement optimal ;
- remplacer des liquides physiologiques déficients.

L'invention concerne également un kit comprenant une composition aqueuse stérilisée selon l'invention, conditionnée en seringues et stérilisées après conditionnement.

L'invention concerne également un kit comprenant une composition aqueuse stérilisée selon l'invention, conditionnée en flacons uni-doses et stérilisée après conditionnement.

EXEMPLES

a) Fabrication des gels

- Gels d'acide hyaluronique non réticulé

Des fibres de hyaluronate de sodium (NaHA) de qualité injectable sont pesées dans un récipient. Une solution aqueuse de tampon phosphate est ajoutée, le tout est homogénéisé pendant environ 1 heure à la spatule, à température ambiante et sous une pression atmosphérique de 900 mmHg.

- Gels d'acide hyaluronique réticulé

Les gels comprenant de l'acide hyaluronique réticulé sont obtenus selon le mode opératoire décrit dans la demande de brevet WO 2009/071697 au nom de la demanderesse à partir de fibres de hyaluronate de sodium (NaHA) et d'éther de butanedioldiglycidyle (BDDE).

- Gels d'acide hyaluronique réticulé et interpénétré

Les gels comprenant de l'acide hyaluronique réticulé et interpénétrés sont obtenus selon le mode opératoire décrit dans la demande de brevet WO 2009/071697 au nom de la demanderesse.

- Gels d'acide hyaluronique co-réticulé

Les gels comprenant de l'acide hyaluronique co-réticulé sont obtenus selon le mode opératoire décrit dans la demande de brevet WO 86/00079 au nom de ALLERGAN.

- Anesthésiques locaux

Les anesthésiques locaux sont solubilisés dans une solution de tampon phosphate stabilisé à un pH proche du pH physiologique avant leur incorporation dans les gels d'acide hyaluronique réticulé ou non réticulé.

- Antioxydants et composés additionnels

Les antioxydants ou les composés additionnels sont solubilisés dans une solution de tampon phosphate avant leur incorporation dans les gels d'acide hyalutronique réticulé ou non réticulé.

- Stérilisation

Les compositions ainsi obtenues sont conditionnées en seringues qui sont stérilisées par autoclavage à la vapeur (T=121°C, 10 min).

b) Mesures des propriétés rhéologiques

Les composantes élastiques G', des compositions comprenant de l'acide hyaluronique réticulé ou non réticulé avant et après stérilisation par autoclavage à la vapeur ont été mesurées sur rhéomètre TA Instrument AR 2000 Ex, en oscillation à 25°C, les valeurs de la composante élastique G' étant relevées à une fréquence de 1 Hz.

La viscosité η des compositions est mesurée sur rhéomètre TA Instruments AR 2000 Ex, en contrainte imposée à 25°C. La valeur de viscosité est relevée à une contrainte de 0,02 s$^{-1}$.

Dans les exemples qui suivent les abréviations utilisées sont les suivantes : AL : Anesthésique local ; Aox : antioxydant ; CA : Composé additionnel ; LIDO : lidocaïne ; MEPI : mépivacaïne ; PRILO : prilocaïne ; HA : acide hyaluronique ; % G' : % d'amélioration de la composante élastique G' par rapport à la composition de référence.

Exemple 1 :

L'exemple 1 illustre l'influence de différents anesthésiques locaux sur l'altération lors de la stérilisation à la chaleur des propriétés rhéologiques de gels d'acide hyaluronique de différentes masses moléculaires, à différentes concentrations non réticulé ou réticulé et à différents taux de réticulation.

Exemple 1-a :

L'exemple 1-a illustre l'influence de différents anesthésiques locaux sur l'altération lors de la stérilisation à la chaleur des propriétés rhéologiques d'un gel d'acide hyaluronique de masse moléculaire moyenne en poids de 3.10$^6$ Da à une concentration de 20 mg/g avec un taux de réticulation X=0,12.

Le ratio [HA]/[MEPI] ou [HA]/[AL] varie de 6,67 à 2.

Pour toutes les mesures, une composition de référence est formulée, en remplaçant la solution aqueuse d'anesthésique local par une quantité équivalente de solution aqueuse de tampon phosphate (les composés additionnels étant conservés).

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence et Y' = Pourcentage de perte à la stérilisation de la composante élastique G' de la composition testée.

Le pourcentage de perte à la stérilisation Y ou Y' de la composante G' est calculé comme suit :
%perte = G' avant stérilisation - G' après stérilisation / G' avant stérilisation

Tableau 1

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 1 | aucun | 0 | aucun | 0 | aucun | 0 | n/a | 0 |
| 2 | LIDO | 3 | aucun | 0 | aucun | 0 | 6,67 | 32 |
| 3 | LIDO | 6 | aucun | 0 | aucun | 0 | 3,33 | 13 |
| 4 | LIDO | 10 | aucun | 0 | aucun | 0 | 2 | -4 |
| 5 | MEPI | 3 | aucun | 0 | aucun | 0 | 6,67 | 34 |
| 6 | MEPI | 6 | aucun | 0 | aucun | 0 | 3,33 | 26 |
| 7 | MEPI | 10 | aucun | 0 | aucun | 0 | 2 | 24 |
| 8 | PRILO | 3 | aucun | 0 | aucun | 0 | 6,67 | 25 |
| 9 | PRILO | 6 | aucun | 0 | aucun | 0 | 3,33 | 2 |

A concentration équivalentes, les propriétés rhéologiques lors de la stérilisation à la chaleur de compositions comprenant de l'acide hyaluronique de masse moléculaire moyenne en poids de 3.10$^6$ Da à une concentration de 20 mg/g avec un taux de réticulation X=0,12 sont moins altérées en présence de mépivacaïne que de lidocaïne ou de prilocaïne et ce quel que soit le ratio.

Exemple 1-b :

L'exemple 1-b illustre l'influence de différents anesthésiques locaux sur l'altération lors de la stérilisation à la chaleur des propriétés rhéologiques d'un gel d'acide hyaluronique de masse moléculaire moyenne en poids de $1.10^6$ Da à une concentration de 20 mg/g avec un taux de réticulation de X= 0,07.

Le ratio [HA]/[MEPI] ou [HA]/[AL] varie de 6,67 à 3,33.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 2 ci-dessous recense les différentes compositions testées et les résultats obtenus. Les abréviations utilisées sont les suivantes : AL : Anesthésique local ; Aox : antioxydant ; CA : Composé additionnel ; LIDO : lidocaïne ; MEPI : mépivacaïne ; PRILO : prilocaïne ; HA : acide hyaluronique ; % G' : % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence et Y' = Pourcentage de perte à la stérilisation de la composante élastique G' de la composition testée.

Tableau 2

| N° | AL | | Aox | | CA | | [HA]/[MEPI] ou [HA]/[AL] | % G' |
|----|------|-----------|-------|------------|-------|-----------|--------------------------|------|
|    | nature | [AL] (mg/g) | nature | [Aox] (mg/g) | nature | [CA] (mg/g) | | |
| 10 | aucun | 0 | aucun | 0 | aucun | 0 | n/a | 0 |
| 11 | LIDO | 3 | aucun | 0 | aucun | 0 | 6,67 | 37 |
| 12 | LIDO | 6 | aucun | 0 | aucun | 0 | 3,33 | 28 |
| 13 | MEPI | 3 | aucun | 0 | aucun | 0 | 6,67 | 41 |
| 14 | MEPI | 6 | aucun | 0 | aucun | 0 | 3,33 | 40 |
| 15 | PRILO | 3 | aucun | 0 | aucun | 0 | 6,67 | 26 |

Comme à l'exemple 1-a, mais en présence d'acide hyaluronique de plus faible masse moléculaire moyenne en poids et à un taux de réticulation plus faible, les résultats obtenus sont confirmés quel que soit le ratio.

Comme les meilleurs résultats ont été obtenus en présence de lidocaïne ou de mépivacaïne, l'étude sera poursuivie en comparant les résultats obtenus uniquement en présence de mépivacaïne ou de lidocaïne.

Exemple 1-c :

L'exemple 1-c illustre l'influence de différents anesthésiques sur l'altération lors de la stérilisation à la chaleur des propriétés rhéologiques d'un gel d'acide hyaluronique de masse moléculaire moyenne en poids de $3.10^6$ Da à une concentration de 20 mg/g avec un taux de réticulation X=0,06.

Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 3 ci-dessous recense les différentes compositions testées et les résultats obtenus. Les abréviations utilisées sont les suivantes : AL : Anesthésique local ; Aox : antioxydant ; CA : Composé additionnel ; LIDO : lidocaïne ; MEPI : mépivacaïne ; HA : acide hyaluronique% ; G' : % d'amélioration de la composante élastique G' par rapport à la composition de référence.

Le pourcentage d'amélioration de la composante élastique G' est défini comme étant :

$$\text{\% d'amélioration } G' = 100 * (Y-Y')/Y$$

avec Y = Pourcentage de perte à la stérilisation de la composante élastique G' de la composition de référence
et Y' = Pourcentage de perte à la stérilisation de la composante élastique G' de la composition testée.

Tableau 3

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 16 | aucun | 0 | aucun | 0 | aucun | 0 | n/a | 0 |
| 17 | LIDO | 3 | aucun | 0 | aucun | 0 | 6,67 | -1 |
| 18 | MEPI | 3 | aucun | 0 | aucun | 0 | 6,67 | 12 |

Les résultats obtenus à l'exemple 1-a sont confirmés avec de l'acide hyaluronique de même masse moléculaire moyenne en poids, à la même concentration mais avec un taux de réticulation inférieur. Les résultats obtenus à l'exemple 1-b sont également confirmés avec de l'acide hyaluronique de poids moléculaire moyen supérieur, à la même concentration et à un taux de réticulation comparable.

Exemple 1-d :

L'exemple 1-d illustre l'influence de différents anesthésiques locaux sur l'altération lors de la stérilisation à la chaleur des propriétés rhéologiques d'un gel d'acide hyaluronique de masse moléculaire moyenne en poids de $3.10^6$ Da à une concentration de 20 mg/g, non réticulé.
Le ratio [HA]/[MEPI] ou [HA]/[AL] varie de 20 à 2.
Pour toutes les mesures, une composition de référence est formulée, en remplaçant la solution aqueuse d'anesthésique local par une quantité équivalente de solution aqueuse de tampon phosphate (les composés additionnels étant conservés).
Le tableau 4 ci-dessous recense les différentes compositions testées et les résultats obtenus. Les abréviations utilisées sont les suivantes : AL : Anesthésique local ; Aox : antioxydant ; CA : Composé additionnel ; LIDO : lidocaïne ; MEPI : mépivacaïne ; HA : acide hyaluronique ; % $\eta$ : % d'amélioration de la viscosité $\eta$ par rapport à la composition de référence.
Le pourcentage d'amélioration de la viscosité $\eta$ est défini comme étant :

$$\text{\% d'amélioration } \eta = 100 * (Z-Z')/Z$$

avec Z = Pourcentage de perte à la stérilisation de la viscosité $\eta$ de la composition de référence
et Z' = Pourcentage de perte à la stérilisation de la viscosité $\eta$ de la composition testée.

Tableau 4

| N° | AL | | Aox | | CA | | [HA]/[MEPI] ou [HA]/[AL] | % $\eta$ |
|---|---|---|---|---|---|---|---|---|
| | nature | [AL] (mg/g) | nature | [Aox] (mg/g) | nature | [CA] (mg/g) | | |
| 19 | aucun | 0 | aucun | 0 | aucun | 0 | n/a | 0 |
| 20 | LIDO | 1 | aucun | 0 | aucun | 0 | 20 | -29 |
| 21 | LIDO | 3 | aucun | 0 | aucun | 0 | 6,67 | -44 |
| 22 | LIDO | 6 | aucun | 0 | aucun | 0 | 3,33 | -48 |
| 23 | LIDO | 10 | aucun | 0 | aucun | 0 | 2 | -49 |
| 24 | MEPI | 1 | aucun | 0 | aucun | 0 | 20 | -21 |
| 25 | MEPI | 3 | aucun | 0 | aucun | 0 | 6,67 | -37 |

(suite)

| N° | AL | | Aox | | CA | | [HA]/[MEPI] ou [HA]/[AL] | % η |
|----|------|----------|-------|------------|--------|-----------|--------------------------|-----|
| | nature | [AL] (mg/g) | nature | [Aox] (mg/g) | nature | [CA] (mg/g) | | |
| 26 | MEPI | 6 | aucun | 0 | aucun | 0 | 3,33 | -44 |
| 27 | MEPI | 10 | aucun | 0 | aucun | 0 | 2 | -46 |

Les résultats obtenus aux exemples 1-a et 1-c sont confirmés avec de l'acide hyaluronique de même masse moléculaire moyenne en poids à la même concentration mais non réticulé quel que soit le ratio.

Les propriétés rhéologiques lors de la stérilisation à la chaleur de compositions comprenant de l'acide hyaluronique à une concentration de 20 mg/g sont moins altérées en présence de mépivacaïne que de lidocaïne ou de prilocaïne, à concentration équivalentes, et ce quels que soient le ratio, la masse moléculaire moyenne en poids et le taux de réticulation.

Exemple 2 :

L'exemple 2 illustre l'influence de différents anesthésiques locaux en présence d'un antioxydant sur l'altération lors de la stérilisation à la chaleur des propriétés rhéologiques de gels d'acide hyaluronique de différentes masses moléculaires, à différentes concentrations non réticulé ou réticulé et à différents taux de réticulation.

Exemple 2-a :

L'exemple 2-a illustre l'influence de différents anesthésiques locaux en présence de mannitol sur l'altération lors de la stérilisation à la chaleur des propriétés rhéologiques d'un gel d'acide hyaluronique de masse moléculaire moyenne en poids de $3.10^6$ Da avec un taux de réticulation X =0,12 à une concentration de 20 mg/g.

Le ratio [HA]/[MEPI] ou [HA]/[AL] varie de 20 à 2.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 5 ci-dessous recense les différentes compositions testées et les résultats obtenus. Les abréviations utilisées sont les suivantes : **AL** : Anesthésique local ; Aox : antioxydant ; MAN : mannitol ; CA : Composé additionnel ; LIDO : lidocaïne ; MEPI : mépivacaïne ; PRILO : prilocaïne ; HA : acide hyaluronique % G' : % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence et Y' = Pourcentage de perte à la stérilisation de la composante élastique G' de la composition testée.

Tableau 5

| N° | AL | | Aox | | CA | | [HA]/[MEPI] ou [HA]/[AL] | % G' |
|----|-------|----------|-------|------------|--------|-----------|--------------------------|------|
| | nature | [AL] (mg/g) | nature | [Aox] (mg/g) | nature | [AL] (mg/g) | | |
| 28 | aucun | 0 | MAN | 35 | aucun | 0 | n/a | 0 |
| 29 | LIDO | 1 | MAN | 35 | aucun | 0 | 20 | 11 |
| 30 | LIDO | 3 | MAN | 35 | aucun | 0 | 6,67 | -36 |
| 31 | LIDO | 6 | MAN | 35 | aucun | 0 | 3,33 | -85 |
| 32 | LIDO | 10 | MAN | 35 | aucun | 0 | 2 | -96 |
| 33 | MEPI | 1 | MAN | 35 | aucun | 0 | 20 | 19 |
| 34 | MEPI | 3 | MAN | 35 | aucun | 0 | 6,67 | -7 |

(suite)

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [AL] (mg/g) | | |
| 35 | MEPI | 6 | MAN | 35 | aucun | 0 | 3,33 | -34 |
| 36 | MEPI | 10 | MAN | 35 | aucun | 0 | 2 | -50 |
| 37 | PRILO | 3 | MAN | 35 | aucun | 0 | 6,67 | -60 |
| 38 | PRILO | 6 | MAN | 35 | aucun | 0 | 3,33 | -92 |

A concentrations équivalentes en présence de mannitol, les propriétés rhéologiques lors de la stérilisation à la chaleur de compositions comprenant de l'acide hyaluronique de masse moléculaire moyenne en poids de $3.10^6$ Da à une concentration de 20 mg/g et avec un taux de réticulation X=0,12 sont moins altérées en présence de mépivacaïne que de lidocaïne ou de prilocaïne et ce quel que soit le ratio.

Exemple 2-b :

L'exemple 2-b illustre l'influence de différents anesthésiques locaux en présence d'un antioxydant sur l'altération lors de la stérilisation à la chaleur des propriétés rhéologiques d'un gel d'acide hyaluronique de masse moléculaire moyenne en poids de $1.10^6$ Da à une concentration de 20 mg/g, avec un taux de réticulation de X= 0,07 .
Le ratio [HA]/[MEPI] ou [HA]/[AL] varie de 6,67 à 3,33.
Pour toutes les mesures, une composition de référence est formulée, en remplaçant la solution aqueuse d'anesthésique local par une quantité équivalente de solution aqueuse de tampon phosphate (les composés additionnels étant conservés).
Le tableau 6 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; LIDO : lidocaïne ; MEPI : mépivacaïne ; PRILO : prilocaïne ; HA : acide hyaluronique ; % G' : % d'amélioration de la composante élastique G' par rapport à la composition de référence.
Le pourcentage d'amélioration de la composante élastique G' est défini comme étant :

$$\% \ d'amélioration \ G' = 100 * (Y-Y')/Y$$

avec Y = Pourcentage de perte à la stérilisation de la composante élastique G' de la composition de référence et Y' = Pourcentage de perte à la stérilisation de la composante élastique G' de la composition testée.

Tableau 6

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 39 | aucun | 0 | MAN | 35 | aucun | 0 | n/a | 0 |
| 40 | LIDO | 3 | MAN | 35 | aucun | 0 | 6,67 | -49 |
| 41 | LIDO | 6 | MAN | 35 | aucun | 0 | 3,33 | -83 |
| 42 | MEPI | 3 | MAN | 35 | aucun | 0 | 6,67 | -23 |
| 43 | MEPI | 6 | MAN | 35 | aucun | 0 | 3,33 | -52 |
| 44 | PRILO | 3 | MAN | 35 | aucun | 0 | 6,67 | -63 |

Comme à l'exemple 2-a, mais en présence d'acide hyaluronique de plus faible masse moléculaire moyenne en poids et à un taux de réticulation plus faible, les résultats obtenus sont confirmés quel que soit le ratio.
Les meilleurs résultats ayant été obtenus en présence de lidocaïne ou de mépivacaïne, la suite de l'étude a été faite uniquement en comparant les résultats obtenus en présence de mépivacaïne ou de lidocaïne.

Exemple 2-c :

L'exemple 2-c illustre l'influence de différents anesthésiques locaux en présence d'un antioxydant sur l'altération lors de la stérilisation à la chaleur des propriétés rhéologiques d'un gel d'acide hyaluronique de masse moléculaire moyenne en poids de $3.10^6$ Da à une concentration de 20 mg/g avec un taux de réticulation X=0,06.

Le ratio [HA]/[MEPI] ou [HA]/[AL] est de 3,33.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 7 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; LIDO : lidocaïne ; MEPI : mépivacaïne ; HA : acide hyaluronique ; % G' : % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence
et Y' = Pourcentage de perte à la stérilisation de la composante élastique G' de la composition testée.

Tableau 7

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 45 | aucun | 0 | MAN | 35 | aucun | 0 | n/a | 0 |
| 46 | LIDO | 3 | MAN | 35 | aucun | 0 | 3,33 | -168 |
| 47 | MEPI | 3 | MAN | 35 | aucun | 0 | 3,33 | -95 |

Les résultats obtenus à l'exemple 2-a sont confirmés avec de l'acide hyaluronique de même masse moléculaire moyenne en poids, à la même concentration mais avec un taux de réticulation inférieur. Les résultats obtenus à l'exemple 2-b sont également confirmés avec de l'acide hyaluronique de masse moléculaire moyenne en poids supérieure, à la même concentration avec un taux de réticulation voisin.

Exemple 2-d :

L'exemple 2-d illustre l'influence de différents anesthésiques locaux en présence de mannitol sur l'altération lors de la stérilisation à la chaleur des propriétés rhéologiques d'un gel d'acide hyaluronique de masse moléculaire moyenne en poids de $3.10^6$ Da à une concentration de 20 mg/g, non réticulé.

Le ratio [HA]/[MEPI] ou [HA]/[AL] varie de 20 à 2.

Pour toutes les mesures, une composition de référence est formulée, en remplaçant la solution aqueuse d'anesthésique local par une quantité équivalente de solution aqueuse de tampon phosphate.

Le tableau 8 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; MEPI : mépivacaïne, HA, acide hyaluronique; % η : % d'amélioration de la viscosité η par rapport à la composition de référence.

Le pourcentage d'amélioration de la viscosité η est défini comme étant :

$$\% \text{ d'amélioration } \eta = 100 * (Z-Z')/Z$$

avec Y = Pourcentage de perte à la stérilisation de la viscosité η de la composition de référence
et Y' = Pourcentage de perte à la stérilisation de la viscosité η de la composition testée.

Tableau 8

| N° | AL | | Aox | | CA | | [HA]/[MEPI] ou [HA]/[AL] | % η |
|---|---|---|---|---|---|---|---|---|
| | nature | [AL] (mg/g) | nature | [Aox] (mg/g) | nature | [CA] (mg/g) | | |
| 48 | aucun | 0 | MAN | 35 | aucun | 0 | n/a | 0 |
| 49 | LIDO | 1 | MAN | 35 | aucun | 0 | 20 | -50 |
| 50 | LIDO | 3 | MAN | 35 | aucun | 0 | 6,67 | -66 |
| 51 | LIDO | 6 | MAN | 35 | aucun | 0 | 3,33 | -69 |
| 52 | LIDO | 10 | MAN | 35 | aucun | 0 | 2 | -70 |
| 53 | MEPI | 1 | MAN | 35 | aucun | 0 | 20 | -43 |
| 54 | MEPI | 3 | MAN | 35 | aucun | 0 | 6,67 | -62 |
| 55 | MEPI | 6 | MAN | 35 | aucun | 0 | 3,33 | -67 |
| 56 | MEPI | 10 | MAN | 35 | aucun | 0 | 2 | -68 |

Les résultats obtenus aux exemples 2-a et 2-c sont confirmés avec de l'acide hyaluronique de même masse moléculaire moyenne en poids, à la même concentration mais non réticulé quel que soit le ratio.

A concentration équivalentes, les propriétés rhéologiques lors de la stérilisation à la chaleur de compositions comprenant de l'acide hyaluronique à une concentration de 20 mg/g comprenant du mannitol sont moins altérées en présence de mépivacaïne que de lidocaïne ou de prilocaïne et ce quels que soient le ratio, la masse moléculaire moyenne en poids et le taux de réticulation.

Exemple 3 :

L'exemple 3 illustre l'influence de différents anesthésiques locaux en présence d'un sel d'ascorbyl phosphate de magnésium, ci-après MAP sur l'altération lors de la stérilisation à la chaleur des propriétés rhéologiques de gels d'acide hyaluronique à différents taux de réticulation.

Exemple 3-a

L'exemple 3-a illustre l'influence de différents anesthésiques locaux en présence de MAP sur l'altération lors de la stérilisation à la chaleur des propriétés rhéologiques d'un gel d'acide hyaluronique de masse moléculaire moyenne en poids de $3.10^6$ Da avec un taux de réticulation X=0,12 à une concentration de 20 mg/g.

Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 9 ci-dessous recense les différentes compositions testées et les résultats obtenus. Les abréviations utilisées sont les suivantes : AL : Anesthésique local ; Aox : antioxydant ; CA : Composé additionnel ; MAP : ascorbyl phosphate de magnésium ; LIDO : lidocaïne ; MEPI : mépivacaïne ; HA : acide hyaluronique ; % G' : % d'amélioration de la composante élastique G' par rapport à la composition de référence.

Le pourcentage d'amélioration de la composante élastique G' est défini comme étant :

$$\% \text{ d'amélioration G'} = 100 * (Y-Y')/Y$$

avec Y = Pourcentage de perte à la stérilisation de la composante élastique G' de la composition de référence et Y' = Pourcentage de perte à la stérilisation de la composante élastique G' de la composition testée.

Tableau 9

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 57 | aucun | 0 | aucun | 0 | MAP | 0,3 | n/a | 0 |
| 58 | LIDO | 3 | aucun | 0 | MAP | 0,3 | 6,67 | -42 |
| 59 | MEPI | 3 | aucun | 0 | MAP | 0,3 | 6,67 | -28 |

En présence MAP, les propriétés rhéologiques lors de la stérilisation à la chaleur de compositions comprenant de l'acide hyaluronique de masse moléculaire moyenne en poids de $3.10^6$ Da avec un taux de réticulation X=0,06 et à une concentration de 20 mg/g sont moins altérées en présence de mépivacaïne que de lidocaïne, à concentrations équivalente, à un ratio [HA]/[MEPI] de 6,67.

Exemple 3-b

L'exemple 3-b illustre l'influence de différents anesthésiques locaux en présence de MAP sur l'altération lors de la stérilisation à la chaleur des propriétés rhéologiques d'un gel d'acide hyaluronique de masse moléculaire moyenne en poids de $3.10^6$ Da avec un taux de réticulation X=0,06 à une concentration de 20 mg/g.
Le ratio [HA]/[MEPI] ou [HA]/[AL] est de 6,67.
Pour toutes les mesures, une composition de référence est formulée, en remplaçant la solution aqueuse d'anesthésique local par une quantité équivalente de solution aqueuse de tampon phosphate (les composés additionnels étant conservés).
Le tableau 10 ci-dessous recense les différentes compositions testées et les résultats obtenus. Les abréviations utilisées sont les suivantes : AL : Anesthésique local ; Aox : antioxydant ; CA : Composé additionnel ; MAP : ascorbyl phosphate de magnésium ; LIDO : lidocaïne ; MEPI : mépivacaïne ; HA : acide hyaluronique ; % G' : % d'amélioration de la composante élastique G' par rapport à la composition de référence.
Le pourcentage d'amélioration de la composante élastique G' est défini comme étant :

$$\% \text{ d'amélioration G'} = 100 * (Y-Y')/Y$$

avec Y = Pourcentage de perte à la stérilisation de la composante élastique G' de la composition de référence
et Y' = Pourcentage de perte à la stérilisation de la composante élastique G' de la composition testée.

Tableau 10

| N° | AL Aox | | | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 60 | aucun | 0 | aucun | 0 | MAP | 0,3 | n/a | 0 |
| 61 | LIDO | 3 | aucun | 0 | MAP | 0,3 | 6,67 | -42 |
| 62 | MEPI | 3 | aucun | 0 | MAP | 0,3 | 6,67 | -29 |

Les résultats obtenus à l'exemple 3-a sont confirmés avec de l'acide hyaluronique de même masse moléculaire moyenne en poids, à la même concentration et au même ratio [HA]/[MEPI], mais à un taux de réticulation inférieur.
Les propriétés rhéologiques lors de la stérilisation à la chaleur de compositions comprenant de l'acide hyaluronique à une concentration de 20 mg/g en présence de MAP sont moins altérées en présence de mépivacaïne que de lidocaïne à concentration équivalente, et ce quel que soit le taux de réticulation.

Exemple 4 :

L'exemple 4 illustre l'influence de différents anesthésiques locaux en présence de mannitol et de SOS sur l'altération lors de la stérilisation à la chaleur des propriétés rhéologiques de gels d'acide hyaluronique de masse moléculaire moyenne en poids de $3.10^6$ Da avec un taux de réticulation X=0,12 à une concentration de 20 mg/g.

Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 11 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; SOS : sucrose octasulfate ; LIDO : lidocaïne ; MEPI : mépivacaïne ; HA : acide hyaluronique ; % G' : % d'amélioration de la composante élastique G' par rapport à la composition de référence.

Le pourcentage d'amélioration de la composante élastique G' est défini comme étant :

$$\% \ d'amélioration \ G' = 100 * (Y-Y')/Y$$

avec Y = Pourcentage de perte à la stérilisation de la composante élastique G' de la composition de référence,
et Y' = Pourcentage de perte à la stérilisation de la composante élastique G' de la composition testée.

Tableau 11

| N° | AL | | Aox | | CA | | [HA]/[MEPI] ou [HA]/[AL] | % G' |
|----|------|-----------|------|------------|------|-----------|--------------------------|------|
| | nature | [AL] (mg/g) | nature | [Aox] (mg/g) | nature | [CA] (mg/g) | | |
| 63 | aucun | 0 | MAN | 35 | SOS | 1 | n/a | 0 |
| 64 | LIDO | 3 | MAN | 35 | SOS | 1 | 6,67 | -21 |
| 65 | MEPI | 3 | MAN | 35 | SOS | 1 | 6,67 | -14 |

A concentration équivalentes, en présence de mannitol et de SOS les propriétés rhéologiques lors de la stérilisation à la chaleur de compositions comprenant de l'acide hyaluronique à une concentration de 20 mg/g sont moins altérées en présence de mépivacaïne que de lidocaïne à un ratio de 6,67.

Exemple 5

L'exemple 5 illustre l'influence de différents anesthésiques locaux en présence de mannitol et de MAP sur l'altération lors de la stérilisation à la chaleur des propriétés rhéologiques de gels d'acide hyaluronique de masse moléculaire moyenne en poids de $3.10^6$ Da avec un taux de réticulation X=0,12 à une concentration de 20 mg/g.

Exemple 5-a

L'exemple 5-a illustre l'influence de différents anesthésiques locaux en présence de mannitol et de MAP à une concentration de 0,3 mg/g, sur l'altération lors de la stérilisation à la chaleur des propriétés rhéologiques de gels d'acide hyaluronique de masse moléculaire moyenne en poids de $3.10^6$ Da avec un taux de réticulation X=0,12 à une concentration de 20 mg/g.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 12 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; MAP : ascorbyl phosphate de magnésium ; LIDO : lidocaïne ; MEPI : mépivacaïne ; HA : acide hyaluronique ; % G' : % d'amélioration de la composante élastique G' par rapport à la composition de référence.

Le pourcentage d'amélioration de la composante élastique G' est défini comme étant :

$$\% \ d'amélioration \ G' = 100 * (Y-Y')/Y$$

avec Y = Pourcentage de perte à la stérilisation de la composante élastique G' de la composition de référence,
et Y' = Pourcentage de perte à la stérilisation de la composante élastique G' de la composition testée.

Tableau 12

| N° | AL | | Aox | | CA | | [HA]/[MEPI] ou [HA]/[AL] | % G' |
|---|---|---|---|---|---|---|---|---|
| | nature | [AL] (mg/g) | [Aox] nature (mg/g) | | nature | [CA] (mg/g) | | |
| 66 | aucun | 0 | MAN | 35 | MAP | 0,3 | n/a | 0 |
| 67 | LIDO | 3 | MAN | 35 | MAP | 0,3 | 6,67 | -30 |
| 68 | MEPI | 3 | MAN | 35 | MAP | 0,3 | 6,67 | -20 |

A concentration équivalentes, les propriétés rhéologiques lors de la stérilisation à la chaleur de compositions comprenant de l'acide hyaluronique à une concentration de 20 mg/g en présence de mannitol et de MAP à une concentration de 0,3 mg/g sont moins altérées en présence de mépivacaïne que de lidocaïne à un ratio de 6,67.

Exemple 5-b

L'exemple 5-b illustre l'influence de différents anesthésiques locaux en présence de mannitol et de MAP à une concentration de 0,7 mg/g, sur l'altération lors de la stérilisation à la chaleur des propriétés rhéologiques de gels d'acide hyaluronique de masse moléculaire moyenne en poids de $3.10^6$ Da avec un taux de réticulation X=0,12 à une concentration de 20 mg/g.

Pour toutes les mesures, une composition de référence est formulée, en remplaçant la solution aqueuse d'anesthésique local par une quantité équivalente de solution aqueuse de tampon phosphate (les composés additionnels étant conservés)

Le tableau 13 ci-dessous recense les différentes compositions testées et les résultats obtenus. Les abréviations utilisées sont les suivantes : AL : Anesthésique local ; Aox : antioxydant ; MAN : mannitol ; CA : Composé additionnel ; MAP : ascorbyl phosphate de magnésium ; LIDO : lidocaïne ; MEPI : mépivacaïne ; HA : acide hyaluronique ; % G' : % d'amélioration de la composante élastique G' par rapport à la composition de référence.

Le pourcentage d'amélioration de la composante élastique G' est défini comme étant :

$$\text{\% d'amélioration } G' = 100 * (Y-Y')/Y$$

avec Y = Pourcentage de perte à la stérilisation de la composante élastique G' de la composition de référence, et Y' = Pourcentage de perte à la stérilisation de la composante élastique G' de la composition testée.

Tableau 13

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 69 | aucun | 0 | MAN | 35 | MAP | 0,7 | n/a | 0 |
| 70 | LIDO | 3 | MAN | 35 | MAP | 0,7 | 6,67 | -29 |
| 71 | MEPI | 3 | MAN | 35 | MAP | 0.7 | 6,67 | -17 |

Les résultats obtenus à l'exemple 5-a sont confirmés avec une concentration en MAP supérieure.

Exemple 6

L'exemple 6 permet de comparer la cinétique de relargage de la mépivacaïne à la cinétique de relargage de la lidocaïne introduites chacune dans un gel d'acide hyaluronique de masse moléculaire moyenne en poids de $3.10^6$ Da avec un taux de réticulation de X = 0,12 à une concentration de 20 mg/g. Les concentrations initiales en mépivacaïne ou en lidocaïne sont de 3 mg/g.

Le protocole d'étude des cinétiques de relargage des deux anesthésiques locaux est le même que celui mis en œuvre à l'exemple 5 de la demande de brevet WO 2010/015901 au nom d'Allergan. La cinétique de relargage a cependant été étudiée à 37°C en milieu sérum physiologique. Un suivi par spectrophotométrie UV-Visible est réalisé pour doser l'anesthésique local présent dans le gel.

Dans le tableau 13 ci-dessous les pourcentages massiques de lidocaïne ou de mépivacaïne au sein du gel d'acide hyaluronique mesurés après différents temps de dialyse sont précisés.

Tableau 14

| Temps (h) | Lidocaïne | Mépivacaïne |
|---|---|---|
| 0 | 0,293 | 0,290 |
| 1,5 | 0,160 | 0,133 |
| 5 | 0,109 | 0,100 |
| 7 | 0,103 | 0,098 |
| 23 | 0,091 | 0,086 |
| 48 | 0,084 | 0,077 |
| 72 | 0,082 | 0,075 |

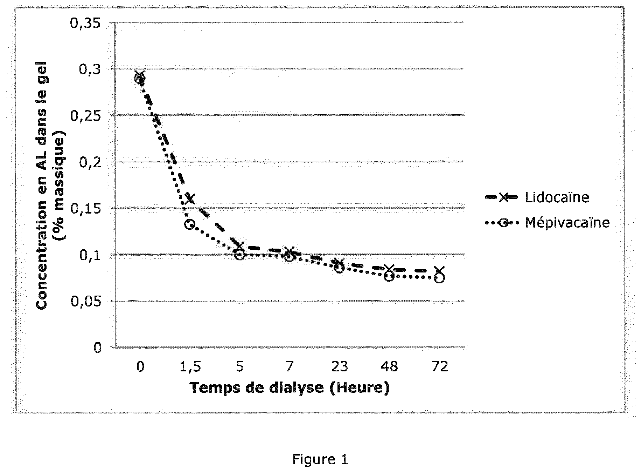

Les résultats ci-dessus obtenus sont illustrés par la Figure 1 qui est un graphique représentant les concentrations en lidocaïne et en mépivacaïne en fonction du temps de dialyse.

La Figure 1 qui représente la concentration massique en anesthésique local (lidocaïne et/ou mépivacaïne) en fonction du temps de dialyse en heure, montre que la cinétique de relargage de la lidocaïne et celle de la mépivacaïne sont comparables.

Ainsi, la quantité biodisponible d'anesthésique local est équivalente que l'on incorpore de la lidocaïne ou que l'on incorpore de la mépivacaïne.

Exemple 7

L'exemple 7 illustre l'influence de différents anesthésiques locaux sur l'altération lors de la stérilisation à la chaleur des propriétés rhéologiques d'un gel d'acide hyaluronique interpénétré réalisé selon la demande de brevet WO 2009/071697 au nom de VIVACY, et comprenant ou non du mannitol.

Exemple 7-a

L'exemple 7-a illustre l'influence de différents anesthésiques locaux sur l'altération lors de la stérilisation à la chaleur des propriétés rhéologiques d'un gel d'acide hyaluronique interpénétré (ne comprenant pas de mannitol) réalisé selon la demande de brevet WO 2009/071697 au nom de VIVACY, à une concentration finale de 20 mg/g, le premier gel étant réticulé avec du NaHA de masse moléculaire moyenne en poids de $1.10^6$ Da avec un taux de réticulation de X= 0,03, et le second gel étant réticulé avec du NaHA de masse moléculaire moyenne en poids de $3.10^6$ Da avec un taux de réticulation X=0,06. Le ratio de mélange des 2 gels étant de 50/50. Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 15 ci-dessous recense les différentes compositions testées et les résultats obtenus. Les abréviations utilisées sont les suivantes : AL: Anesthésique local ; Aox: antioxydant ; CA : Composé additionnel ; LIDO : lidocaïne ; MEPI : mépivacaïne ; HA: acide hyaluronique% ; G': % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence, et Y' = Pourcentage de perte à la stérilisation de la composante élastique G' de la composition testée.

Tableau 15

| N° | AL | | Aox | | CA | | [HA]/[MEPI] ou [HA]/[AL] | % G' |
|----|------|-----------|--------|------------|--------|-----------|--------------------------|------|
| | nature | [AL] (mg/g) | nature | [Aox] (mg/g) | nature | [CA] (mg/g) | | |
| 72 | aucun | 0 | NA | 0 | aucun | 0 | n/a | 0 |
| 73 | LIDO | 3 | NA | 0 | aucun | 0 | 6,67 | -9 |
| 74 | MEPI | 3 | NA | 0 | aucun | 0 | 6,67 | 8 |

A concentrations équivalentes, les propriétés rhéologiques lors de la stérilisation à la chaleur de compositions comprenant de l'acide hyaluronique réticulé et interpénétré réalisées selon la demande de brevet WO 2009/071697 au nom de VIVACY sont moins altérées en présence de mépivacaïne que de lidocaïne.

Exemple 7-b

L'exemple 7-b illustre l'influence de différents anesthésiques locaux sur l'altération lors de la stérilisation à la chaleur des propriétés rhéologiques d'un gel d'acide hyaluronique interpénétré comprenant du mannitol réalisé selon la demande de brevet WO 2009/071697 au nom de VIVACY, à une concentration finale de 20 mg/g, le premier gel étant réticulé avec du NaHA de masse moléculaire moyenne en poids de $1.10^6$ Da avec un taux de réticulation de X= 0,03, et le second gel étant réticulé avec du NaHA de masse moléculaire moyenne en poids de $3.10^6$ Da avec un taux de réticulation X=0,06. Le ratio de mélange des 2 gels étant de 50/50. Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 16 ci-dessous recense les différentes compositions testées et les résultats obtenus. Les abréviations utilisées sont les suivantes : AL: Anesthésique local ; Aox: antioxydant ; CA : Composé additionnel ; LIDO : lidocaïne ; MEPI : mépivacaïne ; HA: acide hyaluronique% ; G': % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence, et Y' = Pourcentage de perte à la stérilisation de la composante élastique G' de la composition testée.

Tableau 16

| N° | AL | | Aox | | CA | | [HA]/[MEPI] ou [HA]/[AL] | % G' |
|----|------|-----------|----------|------------|--------|-----------|--------------------------|------|
| | nature | [AL] (mg/g) | nature | [Aox] (mg/g) | nature | [CA] (mg/g) | | |
| 75 | aucun | 0 | mannit ol | 35 | aucun | 0 | n/a | 0 |
| 76 | LIDO | 3 | mannit ol | 35 | aucun | 0 | 6,67 | -131 |
| 77 | MEPI | 3 | mannit ol | 35 | aucun | 0 | 6,67 | -102 |

A concentration équivalentes, les propriétés rhéologiques lors de la stérilisation à la chaleur de compositions comprenant de l'acide hyaluronique réticulé et interpénétré réalisées selon la demande de brevet WO 2009/071697 au nom de VIVACY et du mannitol sont moins altérées en présence de mépivacaïne que de lidocaïne.

Exemple 8

L'exemple 8 illustre l'influence de différents anesthésiques locaux (avec et sans mannitol) sur l'altération lors de la stérilisation à la chaleur des propriétés rhéologiques d'un gel d'acide hyaluronique co-réticulé réalisé selon la demande de brevet WO 86/00079 au nom de ALLERGAN.

Exemple 8-a

L'exemple 8-a illustre l'influence de différents anesthésiques locaux (sans mannitol) sur l'altération lors de la stérilisation à la chaleur des propriétés rhéologiques d'un gel d'acide hyaluronique co-réticulé à une concentration finale de 20 mg/g, réalisé suivant la demande de brevet WO 86/00079 au nom de ALLERGAN : durant la réticulation (NaOH 1%, taux de réticulation X = 0,09, 50°C-2h30), 2 masses moléculaires de NaHA sont mélangés en proportion 50/50 : le premier étant de masse moléculaire moyenne en poids de $1.10^6$ Da, et le second de masse moléculaire moyenne en poids de $3.10^6$ Da. Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 17 ci-dessous recense les différentes compositions testées et les résultats obtenus. Les abréviations utilisées sont les suivantes : AL : Anesthésique local ; Aox: antioxydant ; CA : Composé additionnel ; LIDO : lidocaïne ; MEPI : mépivacaïne ; HA: acide hyaluronique% ; G': % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence, et Y' = Pourcentage de perte à la stérilisation de la composante élastique G' de la composition testée.

Tableau 17

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 78 | aucun | 0 | NA | 0 | aucun | 0 | n/a | 0 |
| 79 | LIDO | 3 | NA | 0 | aucun | 0 | 6,67 | 22 |
| 80 | MEPI | 3 | NA | 0 | aucun | 0 | 6,67 | 26 |

A concentration équivalentes, les propriétés rhéologiques lors de la stérilisation à la chaleur de compositions comprenant de l'acide hyaluronique co-réticulé réalisées selon la demande de brevet WO 86/00079 au nom de ALLERGAN sont moins altérées en présence de mépivacaïne que de lidocaïne.

Exemple 8-b

L'exemple 8-b illustre l'influence de différents anesthésiques locaux (avec mannitol) sur l'altération lors de la stérilisation à la chaleur des propriétés rhéologiques d'un gel d'acide hyaluronique co-réticulé à une concentration finale de 20 mg/g, réalisé suivant la demande de brevet WO 86/00079 au nom de ALLERGAN : durant la réticulation (NaOH 1%, taux de réticulation X = 0,09, 50°C-2h30), 2 masses moléculaires de NaHA sont mélangés en proportion 50/50 : le premier étant de masse moléculaire moyenne en poids de $1.10^6$ Da, et le second de masse moléculaire moyenne en poids de $3.10^6$ Da. Le ratio [HA]/[MEPI] ou [HA]/[AL] est de 6,67.

Pour toutes les mesures, une composition de référence est formulée, en remplaçant la solution aqueuse d'anesthésique local par une quantité équivalente de solution aqueuse de tampon phosphate (les composés additionnels étant conservés).

Le tableau 18 ci-dessous recense les différentes compositions testées et les résultats obtenus. Les abréviations utilisées sont les suivantes : AL: Anesthésique local ; Aox: antioxydant ; CA : Composé additionnel ; LIDO : lidocaïne ; MEPI : mépivacaïne ; HA: acide hyaluronique% ; G': % d'amélioration de la composante élastique G' par rapport à la composition de référence.

Le pourcentage d'amélioration de la composante élastique G' est défini comme étant :

$$\% \text{ d'amélioration } G' = 100 * (Y-Y')/Y$$

avec Y = Pourcentage de perte à la stérilisation de la composante élastique G' de la composition de référence,
et Y' = Pourcentage de perte à la stérilisation de la composante élastique G' de la composition testée.

Tableau 18

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g ) | nature | [CA] (mg/g) | | |
| 81 | aucun | 0 | mannitol | 35 | aucun | 0 | n/a | 0 |
| 82 | LIDO | 3 | mannitol | 35 | aucun | 0 | 6,67 | -32 |
| 83 | MEPI | 3 | mannitol | 35 | aucun | 0 | 6,67 | -22 |

A concentration équivalentes, les propriétés rhéologiques lors de la stérilisation à la chaleur de compositions comprenant de l'acide hyaluronique co-réticulé réalisées selon la demande de brevet WO 86/00079 au nom de ALLERGAN et du mannitol sont moins altérées en présence de mépivacaïne que de lidocaïne.

Exemple 9

L'exemple 9 illustre l'influence de l'ajout de différents anesthésiques sur l'altération lors de la stérilisation à la chaleur des propriétés rhéologiques de différents gels d'acide hyaluroniques comprenant divers polyols.

Exemple 9-a

L'exemple 9-a consiste en une compilation d'essais relatifs à de l'acide hyaluronique de masse moléculaire moyenne en poids de $3.10^6$ Da avec un taux de réticulation X=0,06 à une concentration de 20 mg/g.
Tous les calculs de %G' sont réalisés en prenant comme référence la composition ne comprenant ni polyol, ni anesthésique local, ni composé additionnel.

Tableau 19

| N° | AL | | Aox | | CA | | [HA]/[MEPI] | |
|---|---|---|---|---|---|---|---|---|
| | nature | [AL] (mg/g) | nature | [Aox] (mg/g) | natur e | [CA] (mg/g) | ou [HA]/[AL] | % G' |
| 16 | aucun | 0 | aucun | 0 | aucun | 0 | n/a | 0 |
| 45 | aucun | 0 | mannitol | 35 | aucun | 0 | n/a | 54 |
| 17 | LIDO | 3 | aucun | 0 | aucun | 0 | 6,67 | -1 |
| 18 | MEPI | 3 | aucun | 0 | aucun | 0 | 6,67 | 12 |
| 46 | LIDO | 3 | mannitol | 35 | aucun | 0 | 6,67 | -23 |
| 47 | MEPI | 3 | mannitol | 35 | aucun | 0 | 6,67 | 11 |

Les propriétés rhéologiques lors de la stérilisation à la chaleur de compositions comprenant de l'acide hyaluronique de masse moléculaire moyenne en poids de $3.10^6$ Da à une concentration de 20 mg/g et avec un taux de réticulation X=0,06 sont moins altérées en présence de mépivacaïne, par rapport à la composition de référence.

Exemple 9-b

L'exemple 9-b consiste en une compilation des essais relatifs à de l'acide hyaluronique de masse moléculaire moyenne en poids de $3,2.10^6$ Da avec un taux de réticulation X=0,06 à une concentration de 20 mg/g.
Tous les calculs de %G' sont réalisés en prenant comme référence la composition ne comprenant ni polyol, ni anesthésique local, ni composé additionnel.

Tableau 20

| N° | AL nature | [AL] (mg/g) | Aox nature | [Aox] (mg/g) | CA natur e | [CA] (mg/g) | [HA]/[MEPI] ou [HA]/[AL] | % G' |
|---|---|---|---|---|---|---|---|---|
| 84 | aucun | 0 | aucun | 0 | aucun | 0 | n/a | 0 |
| 96 | aucun | 0 | mannitol | 35 | aucun | 0 | n/a | 56 |
| 90 | aucun | 0 | glycérol | 35 | aucun | 0 | n/a | 54 |
| 87 | aucun | 0 | sorbitol | 35 | aucun | 0 | n/a | 46 |
| 93 | aucun | 0 | maltitol | 35 | aucun | 0 | n/a | 51 |
| 85 | LIDO | 3 | aucun | 0 | aucun | 0 | 6,67 | -9 |
| 86 | MEPI | 3 | aucun | 0 | aucun | 0 | 6,67 | 5 |
| 97 | LIDO | 3 | mannitol | 35 | aucun | 0 | 6,67 | -1 |
| 91 | LIDO | 3 | glycérol | 35 | aucun | 0 | 6,67 | -2 |
| 88 | LIDO | 3 | sorbitol | 35 | aucun | 0 | 6,67 | -7 |
| 94 | LIDO | 3 | maltitol | 35 | aucun | 0 | 6,67 | -2 |
| 98 | MEPI | 3 | mannitol | 35 | aucun | 0 | 6,67 | 7 |
| 92 | MEPI | 3 | glycérol | 35 | aucun | 0 | 6,67 | 7 |
| 89 | MEPI | 3 | sorbitol | 35 | aucun | 0 | 6,67 | 8 |
| 95 | MEPI | 3 | maltitol | 35 | aucun | 0 | 6,67 | 12 |

Les propriétés rhéologiques lors de la stérilisation à la chaleur de compositions comprenant de l'acide hyaluronique de masse moléculaire moyenne en poids de $3,2.10^6$ Da à une concentration de 20 mg/g et avec un taux de réticulation X=0,06 sont moins altérées en présence de mépivacaïne, par rapport à la composition de référence quel que soit le polyol incorporé dans la composition.

La composition présentant les meilleurs résulats est une composition comprenant du maltitol et de a mépivacaïne.

Exemple 9-c

L'exemple 9-c consiste en une compilation des essais relatifs à de l'acide hyaluronique de masse moléculaire moyenne en poids de $1.10^6$ Da avec un taux de réticulation X=0,07 à une concentration de 20 mg/g.

Tous les calculs de %G' sont réalisés en prenant comme référence la composition ne comprenant ni polyol, ni anesthésique local, ni composé additionnel.

Tableau 21

| N° | AL nature | [AL] (mg/g) | Aox nature | [Aox] (mg/g) | CA natur e | [CA] (mg/g) | [HA]/[MEPI] ou [HA]/[AL] | % G' |
|---|---|---|---|---|---|---|---|---|
| 10 | aucun | 0 | aucun | 0 | aucun | 0 | n/a | 0 |
| 39 | aucun | 0 | mannitol | 35 | aucun | 0 | n/a | 42 |
| 11 | LIDO | 3 | aucun | 0 | aucun | 0 | 6,67 | 37 |
| 13 | MEPI | 3 | aucun | 0 | aucun | 0 | 6,67 | 41 |
| 40 | LIDO | 3 | mannitol | 35 | aucun | 0 | 6,67 | 14 |
| 42 | MEPI | 3 | mannitol | 35 | aucun | 0 | 6,67 | 29 |

Les propriétés rhéologiques lors de la stérilisation à la chaleur de compositions comprenant de l'acide hyaluronique de masse moléculaire moyenne en poids de $1.10^6$ Da à une concentration de 20 mg/g et avec un taux de réticulation X=0,07 sont moins altérées en présence de mépivacaïne, par rapport à la composition de référence.

Exemple 9-d

L'exemple 9-d consiste en une compilation des essais relatifs à de l'acide hyaluronique de masse moléculaire moyenne en poids de $3.10^6$ Da avec un taux de réticulation X=0,12 à une concentration de 20 mg/g.

Tous les calculs de %G' sont réalisés en prenant comme référence la composition ne comprenant ni polyol, ni anesthésique local, ni composé additionnel.

Tableau 22

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | natur e | [CA] (mg/g) | | |
| 1 | aucun | 0 | aucun | 0 | aucun | 0 | n/a | 0 |
| 28 | aucun | 0 | mannitol | 35 | aucun | 0 | n/a | 39 |
| 2 | LIDO | 3 | aucun | 0 | aucun | 0 | 6,67 | 32 |
| 5 | MEPI | 3 | aucun | 0 | aucun | 0 | 6,67 | 34 |
| 30 | LIDO | 3 | mannitol | 35 | aucun | 0 | 6,67 | 17 |
| 34 | MEPI | 3 | mannitol | 35 | aucun | 0 | 6,67 | 35 |
| 57 | aucun | 0 | aucun | 0 | MAP | 0,3 | n/a | 35 |
| 58 | LIDO | 3 | aucun | 0 | MAP | 0,3 | 6,67 | 8 |
| 59 | MEPI | 3 | aucun | 0 | MAP | 0,3 | 6,67 | 17 |
| 66 | aucun | 0 | mannitol | 35 | MAP | 0.3 | n/a | 39 |
| 67 | LIDO | 3 | mannitol | 35 | MAP | 0.3 | 6,67 | 21 |
| 68 | MEPI | 3 | mannitol | 35 | MAP | 0.3 | 6,67 | 27 |
| 69 | aucun | 0 | mannitol | 35 | MAP | 0.7 | n/a | 41 |
| 70 | LIDO | 3 | mannitol | 35 | MAP | 0.7 | 6,67 | 24 |
| 71 | MEPI | 3 | mannitol | 35 | MAP | 0.7 | 6,67 | 31 |
| 63 | aucun | 0 | mannitol | 35 | SOS | 1 | n/a | 40 |
| 64 | LIDO | 3 | mannitol | 35 | SOS | 1 | 6,67 | 27 |
| 65 | MEPI | 3 | mannitol | 35 | SOS | 1 | 6,67 | 31 |

Les propriétés rhéologiques lors de la stérilisation à la chaleur de compositions comprenant de l'acide hyaluronique de masse moléculaire moyenne en poids de $3.10^6$ Da à une concentration de 20 mg/g et avec un taux de réticulation X=0,12 sont moins altérées en présence de mépivacaïne, par rapport à la composition de référence.

Exemple 9-e

L'exemple 9-e consiste en une compilation des essais ci-desssu cités relatifs à de l'acide hyaluronique interpénétré réalisés selon la demande de brevet WO 2009/071697 au nom de VIVACY.

Tous les calculs de %G' sont réalisés en prenant comme référence la composition ne comprenant ni polyol, ni anesthésique local, ni composé additionnel.

Tableau 23

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | natur e | [CA] (mg(g) | | |
| 72 | aucun | 0 | aucun | 0 | aucun | 0 | n/a | 0 |
| 75 | aucun | 0 | mannitol | 35 | aucun | 0 | n/a | 54 |
| 73 | LIDO | 3 | aucun | 0 | aucun | 0 | 6,67 | -9 |

(suite)

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | natur e | [CA] (mg(g) | | |
| 74 | MEPI | 3 | aucun | 0 | aucun | 0 | 6,67 | 8 |
| 76 | LIDO | 3 | mannitol | 35 | aucun | 0 | 6,67 | -4 |
| 77 | MEPI | 3 | mannitol | 35 | aucun | 0 | 6,67 | 12 |

Les propriétés rhéologiques lors de la stérilisation à la chaleur de compositions comprenant de l'acide hyaluronique interpénétré réalisé selon la demande de brevet WO 2009/071697 au nom de VIVACY sont moins altérées en présence de mépivacaïne, par rapport à la composition de référence.

Exemple 9-f

L'exemple 9-f consiste en une compilation des essais ci-dessu cités relatifs à de l'acide hyaluronique co-réticulé réalisé suivant la demande de brevet WO 86/00079 au nom de ALLERGAN.

Tous les calculs de %G' sont réalisés en prenant comme référence la composition ne comprenant ni polyol, ni anesthésique local, ni composé additionnel.

Tableau 24

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | natur e | [CA] (mg/g) | | |
| 78 | aucun | 0 | aucun | 0 | aucun | 0 | n/a | 0 |
| 81 | aucun | 0 | mannitol | 35 | aucun | 0 | n/a | 24 |
| 79 | LIDO | 3 | aucun | 0 | aucun | 0 | 6,67 | 22 |
| 80 | MEPI | 3 | aucun | 0 | aucun | 0 | 6,67 | 26 |
| 82 | LIDO | 3 | mannitol | 35 | aucun | 0 | 6,67 | -1 |
| 83 | MEPI | 3 | mannitol | 35 | aucun | 0 | 6,67 | 7 |

Les propriétés rhéologiques lors de la stérilisation à la chaleur de compositions comprenant de l'acide hyaluronique co-réticulé réalisé suivant la demande de brevet WO 86/00079 au nom de ALLERGAN sont moins altérées en présence de mépivacaïne, par rapport à la composition de référence.

Exemple 10

L'exemple 10 illustre que l'influence de l'ajout de différents anesthésiques sur l'altération lors de la stérilisation à la chaleur des propriétés rhéologiques de gels d'acide hyaluronique est vérifiée sur une plage de rapport [HA]/[MEPI] $\geq$ 0,1 et à tout le moins comprise entre 0.4 et 2500.

Tableau 25

| N° | AL | | Aox | | CA | | [HA]/[MEPI] ou [HA]/[AL] | % G' |
|---|---|---|---|---|---|---|---|---|
| | nature | [AL] (mg/g) | nature | [Aox] (mg/g) | nature | [CA] (mg/g) | | |
| 84 | MEPI | 0,01 | aucun | 0 | aucun | | 2500 | 0 |
| 85 | LIDO | 0,01 | aucun | 0 | aucun | 0 | 2500 | -7 |
| 86 | ARTI | 0,01 | aucun | 0 | aucun | 0 | 2500 | -14 |
| 87 | PRILO | 0,01 | aucun | 0 | aucun | 0 | 2500 | -6 |
| 88 | MEPI | 25 | aucun | 0 | aucun | | 0,4 | 0 |
| 89 | LIDO | 25 | aucun | 0 | aucun | 0 | 0,4 | -34 |

(suite)

| N° | AL | | Aox | | CA | | [HA]/[MEPI] ou [HA]/[AL] | % G' |
|----|------|-----------|--------|-------------|--------|------------|--------------------------|------|
| | nature | [AL] (mg/g) | nature | [Aox] (mg/g) | nature | [CA] (mg/g) | | |
| 90 | ARTI | 25 | aucun | 0 | aucun | 0 | 0,40 | -49 |
| 91 | PRILO | 25 | aucun | 0 | aucun | 0 | 0,40 | -52 |

**Revendications**

1. Composition aqueuse stérilisée, comprenant au moins un acide hyaluronique et au moins de la mépivacaïne, **caractérisée en ce que** le ratio massique entre la concentration en acide hyaluronique [HA] et la concentration en mépivacaïne [MEPI] : [HA]/[MEPI] est supérieur ou égal à 0,1 ; [HA]/[MEPI] ≥ 0,1.

2. Composition aqueuse stérilisée selon la revendication 1, caractériséé en ce que le ratio massique entre la concentration en acide hyaluronique [HA] et la concentration en mépivacaïne [MEPI] : [HA]/[MEPI] est compris entre 0,1 et 50 ; 0,1 ≤ [HA]/[MEPI] ≤ 50.

3. Composition aqueuse stérilisée selon l'une quelconque des revendications précédentes, **caractérisée en ce que** la concentration en mépivacaïne [MEPI] est comprise entre 0,01 mg/g et 50 mg/g.

4. Composition aqueuse stérilisée selon l'une quelconque des revendications précédente, **caractérisée en ce que** la mépivacaïne est choisi dans le groupe comprenant l'hydrochlorure de mépivacaïne racémique, l'hydrochlorure de (R)-mépivacaïne, l'hydrochlorure de (S)-mépivacaïne, la mépivacaïne racémique, la (R)-mépivacaïne et la (S)-mépivacaïne ou l'un de leurs sels pharmaceutiquement acceptables.

5. Composition aqueuse stérilisée selon l'une quelconque des revendications précédentes, **caractérisée en ce que** la concentration en acide hyaluronique [HA] est comprise entre 2 mg/g et 50 mg/g de poids total de ladite composition.

6. Composition aqueuse stérilisée selon l'une quelconque des revendications précédentes, **caractérisée en ce que** la concentration en acide hyaluronique [HA] est de 20 mg/g de poids total de ladite composition.

7. Composition aqueuse stérilisée selon l'une quelconque des revendications précédentes, **caractérisée en ce qu'**elle comprend au moins un acide hyaluronique non réticulé ou l'un de ses sels, seul ou en mélange.

8. Composition aqueuse stérilisée selon l'une quelconque des revendications 1 à 6, **caractérisée en ce qu'**elle comprend au moins un acide hyaluronique réticulé ou l'un de ses sels, seul ou en mélange.

9. Composition aqueuse stérilisée selon l'une quelconque des revendications précédentes, **caractérisée en ce qu'**elle comprend en outre au moins un antioxydant.

10. Composition aqueuse stérilisée selon l'une quelconque des revendications précédentes, **caractérisée en ce qu'**elle comprend en outre au moins un composé additionnel.

11. Procédé de fabrication d'une composition aqueuse stérilisée selon l'une quelconques des revendications précédentes, **caractérisé en ce qu'**il comprend au moins les étapes suivantes :

    - Une étape d'hydratation dans une solution tampon à pH proche du pH physiologique des fibres d'au moins un acide hyaluronique ou l'un de ses sels, seuls ou en mélange, pour obtenir un hydrogel,
    - Une étape d'incorporation de la mépivacaïne en solution aqueuse avec l'hydrogel obtenu à l'étape précédente,
    - Une étape d'homogénéisation, et
    - Une étape de stérilisation.

12. Procédé de fabrication selon la revendication 11, **caractérisé en ce qu'**il compren en outre au moins une étape de réticulation.

13. Procédé d'obtention d'une composition aqueuse stérilisée d'acide hyaluronique comprenant un anesthésique local, ladite composition ayant des propriétés rhéologiques après stérilisation à la chaleur supérieures aux propriétés rhéologiques d'une composition comprenant de la lidocaïne, **caractérisé en ce que** la lidocaïne est substituée par une quantité équivalente, à un même pH, de mépivacaïne.

14. Utilisation de la mépivacaïne en substitution de la lidocaïne en quantité équivalente pour obtenir une composition d'acide hyaluronique comprenant un anesthésique local dont les propriétés rhéologiques après stérilisation à la chaleur sont supérieures aux propriétés rhéologiques d'une même composition d'acide hyaluronique comprenant de la lidocaïne.

15. Utilisation d'une composition aqueuse stérilisée selon l'une quelconque des revendications 1 à 10, pour la formulation d'une composition de comblement de rides, de correction de défauts cutanés ou volumatrice (pommettes, menton, lèvres).

16. Utilisation d'une composition aqueuse stérilisée selon l'une quelconque des revendications 1 à 10, pour la formulation d'une composition injectable dans une articulation en remplacement ou en complément de liquide synovial déficient.

17. Kit comprenant une composition aqueuse stérilisée selon l'une quelconque des revendications 1 à 10, conditionnée en seringues et stérilisée après conditionnement.

18. Composition aqueuse stérilisée selon l'une quelconque des revendications 1 à 10, pour son utilisation en remplacement ou en complément de liquide synovial déficient.

19. Procédé d'amélioration de la résistance à la dégradation des propriétés rhéologiques d'une composition aqueuse injectable d'acide hyaluronique lors de la stérilisation à la chaleur, **caractérisé en ce que** ladite composition comprend de la mépivacaïne.

20. Utilisation de la mépivacaïne pour améliorer la résistance à la dégradation des propriétés rhéologiques d'une composition aqueuse stérilisée injectable d'acide hyaluronique lors de la stérilisation à la chaleur.

Figure 1

Europäisches
Patentamt

European
Patent Office

Office européen
des brevets

## RAPPORT DE RECHERCHE EUROPEENNE

Numéro de la demande

EP 19 20 9272

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | EP 2 484 387 A1 (Q MED AB [SE]) 8 août 2012 (2012-08-08) | 1-10, 13-15 | INV. A61K31/728 |
| Y | * le document en entier * | 1-15 | A61K31/4458 A61K9/00 |
| X | WO 2013/186493 A2 (VIVACY LAB [FR]) 19 décembre 2013 (2013-12-19) | 1-5, 7-10, 13-15 | A61K9/107 A61K47/10 A61K8/49 |
| Y | * le document en entier * | 1-15 | A61K8/73 A61K8/04 |
| X | FR 2 979 539 A1 (TEOXANE [CH]) 8 mars 2013 (2013-03-08) | 1-5, 7-10, 13-15 | A61Q19/08 A61P19/02 A61P17/00 |
| Y | * le document en entier * | 1-15 | A61P23/00 A61P43/00 |
| X | CN 102 805 882 A (BLOOMAGE FREDA BIOPHARM CO LTD) 5 décembre 2012 (2012-12-05) | 1,2,4,7, 8,13-15 | |
| Y | * le document en entier * | 1-15 | |
| Y | US 2005/136122 A1 (SADOZAI KHALID K [US] ET AL) 23 juin 2005 (2005-06-23) * le document en entier * * exemple 21 * | 1-15 | |
| Y,D | US 2010/028437 A1 (LEBRETON PIERRE F [FR]) 4 février 2010 (2010-02-04) * le document en entier * * revendication 25 * | 1-15 | DOMAINES TECHNIQUES RECHERCHES (IPC) A61K A61Q A61P |
| Y,D | FR 2 938 187 A1 (ANTEIS SA [CH]) 14 mai 2010 (2010-05-14) * le document en entier * | 1-15 | |
| X,P | WO 2014/032804 A1 (VIVACY LAB [FR]) 6 mars 2014 (2014-03-06) * le document en entier * | 1-5, 7-10, 13-15 | |

-/--

Le présent rapport a été établi pour toutes les revendications

| Lieu de la recherche | Date d'achèvement de la recherche | Examinateur |
|---|---|---|
| Munich | 27 janvier 2021 | Houyvet-Landriscina |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
........................................................................
& : membre de la même famille, document correspondant

EPO FORM 1503 03.82 (P04C02)

Europäisches
Patentamt

European
Patent Office

Office européen
des brevets

## RAPPORT DE RECHERCHE EUROPEENNE

Numéro de la demande

EP 19 20 9272

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,P | KR 2014 0025117 A (KIM JONG SEO [KR]) 4 mars 2014 (2014-03-04) * le document en entier * ----- | 1,2,4-8, 13-15 | |
| E | US 2015/038457 A1 (BOURDON FRANCOIS [FR] ET AL) 5 février 2015 (2015-02-05) * le document en entier * * page 6, lignes 18-20 * * page 9, ligne 15 - page 11, ligne 8 * * page 12, lignes 7-9 * * exemples 1-3 * ----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| Munich | 27 janvier 2021 | Houyvet-Landriscina |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 19 20 9272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-01-2021

| Document brevet cité au rapport de recherche | | Date de publication | Membre(s) de la famille de brevet(s) | | Date de publication |
|---|---|---|---|---|---|
| EP 2484387 | A1 | 08-08-2012 | AR | 085128 A1 | 11-09-2013 |
| | | | BR | 112013019500 A2 | 10-11-2020 |
| | | | CA | 2825352 A1 | 09-08-2012 |
| | | | CN | 103415307 A | 27-11-2013 |
| | | | EP | 2484387 A1 | 08-08-2012 |
| | | | EP | 2670447 A1 | 11-12-2013 |
| | | | EP | 2954907 A1 | 16-12-2015 |
| | | | ES | 2548187 T3 | 14-10-2015 |
| | | | JP | 6023086 B2 | 09-11-2016 |
| | | | JP | 2014504623 A | 24-02-2014 |
| | | | KR | 20140049967 A | 28-04-2014 |
| | | | MX | 340148 B | 28-06-2016 |
| | | | RU | 2013140182 A | 10-03-2015 |
| | | | US | 2014039061 A1 | 06-02-2014 |
| | | | US | 2019000740 A1 | 03-01-2019 |
| | | | US | 2020261343 A1 | 20-08-2020 |
| | | | WO | 2012104419 A1 | 09-08-2012 |
| WO 2013186493 | A2 | 19-12-2013 | AU | 2013276343 A1 | 04-12-2014 |
| | | | BR | 112014029510 A2 | 27-06-2017 |
| | | | CA | 2876057 A1 | 19-12-2013 |
| | | | CN | 104394934 A | 04-03-2015 |
| | | | EA | 201492208 A1 | 30-04-2015 |
| | | | EP | 2861301 A2 | 22-04-2015 |
| | | | ES | 2728686 T3 | 28-10-2019 |
| | | | FR | 2991876 A1 | 20-12-2013 |
| | | | JP | 6178414 B2 | 09-08-2017 |
| | | | JP | 2015525099 A | 03-09-2015 |
| | | | KR | 20150023468 A | 05-03-2015 |
| | | | MX | 354544 B | 09-03-2018 |
| | | | PL | 2861301 T3 | 24-08-2020 |
| | | | SG | 11201407857S A | 27-02-2015 |
| | | | TR | 201907427 T4 | 21-06-2019 |
| | | | US | 2014005140 A1 | 02-01-2014 |
| | | | WO | 2013186493 A2 | 19-12-2013 |
| FR 2979539 | A1 | 08-03-2013 | AUCUN | | |
| CN 102805882 | A | 05-12-2012 | AUCUN | | |
| US 2005136122 | A1 | 23-06-2005 | AU | 2003300392 A1 | 03-08-2005 |
| | | | AU | 2009200708 A1 | 02-04-2009 |
| | | | BR | 0318680 A | 12-12-2006 |
| | | | CA | 2551121 A1 | 28-07-2005 |
| | | | CN | 1893989 A | 10-01-2007 |
| | | | DK | 1699500 T3 | 07-05-2018 |

Pour tout renseignement concernant cette annexe : voir Journal Officiel de l'Office européen des brevets, No.12/82

page 1 de 5

**ANNEXE AU RAPPORT DE RECHERCHE EUROPEENNE
RELATIF A LA DEMANDE DE BREVET EUROPEEN NO.**

EP 19 20 9272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-01-2021

| Document brevet cité au rapport de recherche | Date de publication | Membre(s) de la famille de brevet(s) | | Date de publication |
|---|---|---|---|---|
| | | DK | 2656833 T3 | 07-05-2018 |
| | | EP | 1699500 A1 | 13-09-2006 |
| | | EP | 2656833 A1 | 30-10-2013 |
| | | JP | 4594866 B2 | 08-12-2010 |
| | | JP | 2007525541 A | 06-09-2007 |
| | | KR | 20060127897 A | 13-12-2006 |
| | | RU | 2351367 C2 | 10-04-2009 |
| | | US | 2005136122 A1 | 23-06-2005 |
| | | WO | 2005067994 A1 | 28-07-2005 |
| | | ZA | 200605025 B | 31-10-2007 |
| US 2010028437 A1 | 04-02-2010 | AU | 2009278883 A1 | 11-02-2010 |
| | | AU | 2009278884 A1 | 11-02-2010 |
| | | BR | PI0917573 B1 | 13-03-2018 |
| | | BR | PI0917588 B1 | 13-03-2018 |
| | | CA | 2732788 A1 | 11-02-2010 |
| | | CA | 2732928 A1 | 11-02-2010 |
| | | CA | 2944734 A1 | 11-02-2010 |
| | | CA | 3023168 A1 | 11-02-2010 |
| | | CN | 102170855 A | 31-08-2011 |
| | | CN | 102170856 A | 31-08-2011 |
| | | CN | 103285423 A | 11-09-2013 |
| | | CY | 1118281 T1 | 28-06-2017 |
| | | CY | 1118821 T1 | 10-01-2018 |
| | | DK | 2323617 T3 | 24-04-2017 |
| | | DK | 2326302 T3 | 14-11-2016 |
| | | DK | 2674147 T3 | 30-03-2020 |
| | | EP | 2323617 A1 | 25-05-2011 |
| | | EP | 2326302 A1 | 01-06-2011 |
| | | EP | 2674147 A1 | 18-12-2013 |
| | | EP | 3205332 A1 | 16-08-2017 |
| | | EP | 3662889 A1 | 10-06-2020 |
| | | ES | 2599763 T3 | 03-02-2017 |
| | | ES | 2622708 T3 | 07-07-2017 |
| | | ES | 2780190 T3 | 24-08-2020 |
| | | HK | 1156883 A1 | 22-06-2012 |
| | | HK | 1189518 A1 | 13-06-2014 |
| | | HU | E031483 T2 | 28-07-2017 |
| | | HU | E031598 T2 | 28-07-2017 |
| | | HU | E048366 T2 | 28-08-2020 |
| | | JP | 5670899 B2 | 18-02-2015 |
| | | JP | 5670900 B2 | 18-02-2015 |
| | | JP | 5808848 B2 | 10-11-2015 |
| | | JP | 2011529762 A | 15-12-2011 |
| | | JP | 2011529763 A | 15-12-2011 |
| | | JP | 2014237718 A | 18-12-2014 |

Pour tout renseignement concernant cette annexe : voir Journal Officiel de l'Office européen des brevets, No.12/82

EPO FORM P0460

page 2 de 5

**ANNEXE AU RAPPORT DE RECHERCHE EUROPEENNE
RELATIF A LA DEMANDE DE BREVET EUROPEEN NO.**

EP 19 20 9272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-01-2021

| Document brevet cité au rapport de recherche | Date de publication | Membre(s) de la famille de brevet(s) | Date de publication |
|---|---|---|---|
| | | JP 2014237719 A | 18-12-2014 |
| | | JP 2016000350 A | 07-01-2016 |
| | | KR 20110040966 A | 20-04-2011 |
| | | KR 20110043730 A | 27-04-2011 |
| | | MX 342613 B | 06-10-2016 |
| | | PL 2323617 T3 | 31-08-2017 |
| | | PL 2326302 T3 | 31-01-2017 |
| | | PL 2674147 T3 | 16-11-2020 |
| | | PT 2323617 T | 24-04-2017 |
| | | PT 2326302 T | 02-11-2016 |
| | | RU 2011107877 A | 10-09-2012 |
| | | RU 2011107878 A | 10-09-2012 |
| | | SG 10201505441T A | 28-08-2015 |
| | | US 2010028437 A1 | 04-02-2010 |
| | | US 2010028438 A1 | 04-02-2010 |
| | | US 2011118206 A1 | 19-05-2011 |
| | | US 2012172328 A1 | 05-07-2012 |
| | | US 2013041038 A1 | 14-02-2013 |
| | | US 2013041039 A1 | 14-02-2013 |
| | | US 2013102563 A1 | 25-04-2013 |
| | | US 2013131011 A1 | 23-05-2013 |
| | | US 2013244970 A1 | 19-09-2013 |
| | | US 2014148406 A1 | 29-05-2014 |
| | | US 2014213546 A1 | 31-07-2014 |
| | | US 2014213547 A1 | 31-07-2014 |
| | | US 2015045321 A1 | 12-02-2015 |
| | | US 2015297790 A1 | 22-10-2015 |
| | | US 2016279296 A1 | 29-09-2016 |
| | | US 2018000992 A1 | 04-01-2018 |
| | | US 2019076579 A1 | 14-03-2019 |
| | | US 2019076580 A1 | 14-03-2019 |
| | | US 2019343990 A1 | 14-11-2019 |
| | | US 2019350832 A1 | 21-11-2019 |
| | | US 2020086009 A1 | 19-03-2020 |
| | | WO 2010015900 A1 | 11-02-2010 |
| | | WO 2010015901 A1 | 11-02-2010 |
| FR 2938187 A1 | 14-05-2010 | BR PI0919155 A2 | 08-12-2015 |
| | | CA 2742099 A1 | 14-05-2010 |
| | | CN 102196805 A | 21-09-2011 |
| | | DK 2676658 T3 | 21-12-2015 |
| | | EP 2349203 A2 | 03-08-2011 |
| | | EP 2676658 A1 | 25-12-2013 |
| | | ES 2443296 T3 | 18-02-2014 |
| | | ES 2556475 T3 | 18-01-2016 |
| | | FR 2938187 A1 | 14-05-2010 |

Pour tout renseignement concernant cette annexe : voir Journal Officiel de l'Office européen des brevets, No.12/82

EPO FORM P0460

page 3 de 5

**EP 3 799 875 A1**

**ANNEXE AU RAPPORT DE RECHERCHE EUROPEENNE**
**RELATIF A LA DEMANDE DE BREVET EUROPEEN NO.**                    EP 19 20 9272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-01-2021

| Document brevet cité au rapport de recherche | Date de publication | Membre(s) de la famille de brevet(s) | Date de publication |
|---|---|---|---|
| | | JP      5626661 B2 | 19-11-2014 |
| | | JP   2012508217 A | 05-04-2012 |
| | | KR  20110084510 A | 25-07-2011 |
| | | PL      2349203 T3 | 30-04-2014 |
| | | PL      2676658 T3 | 29-07-2016 |
| | | RU   2011121426 A | 10-12-2012 |
| | | US   2011201571 A1 | 18-08-2011 |
| | | WO   2010052430 A2 | 14-05-2010 |
| WO 2014032804    A1   06-03-2014 | | AU      2013307648 A1 | 12-03-2015 |
| | | BR 112015004370 A2 | 04-07-2017 |
| | | CA      2881750 A1 | 06-03-2014 |
| | | CN      104582672 A | 29-04-2015 |
| | | CN      106890360 A | 27-06-2017 |
| | | CR      20150174 A | 27-08-2015 |
| | | EP      2890360 A1 | 08-07-2015 |
| | | FR      2994846 A1 | 07-03-2014 |
| | | HK      1208350 A1 | 04-03-2016 |
| | | JP   2015526489 A | 10-09-2015 |
| | | KR  20150046307 A | 29-04-2015 |
| | | MX       354543 B | 09-03-2018 |
| | | RU   2015111247 A | 27-10-2016 |
| | | SG 10201705217U A | 28-07-2017 |
| | | SG 11201501215Y A | 28-05-2015 |
| | | US   2014088037 A1 | 27-03-2014 |
| | | US   2016303024 A1 | 20-10-2016 |
| | | WO   2014032804 A1 | 06-03-2014 |
| KR 20140025117   A   04-03-2014 | | AUCUN | |
| US 2015038457   A1   05-02-2015 | | AU      2014298046 A1 | 10-03-2016 |
| | | BR 112016002171 A2 | 01-08-2017 |
| | | CA      2919792 A1 | 05-02-2015 |
| | | CN      105682657 A | 15-06-2016 |
| | | DK      3027186 T3 | 16-09-2019 |
| | | EP      3027186 A1 | 08-06-2016 |
| | | EP      3574901 A1 | 04-12-2019 |
| | | ES      2743755 T3 | 20-02-2020 |
| | | HR    P20191345 T1 | 01-11-2019 |
| | | HU     E045969 T2 | 28-01-2020 |
| | | JP      6543625 B2 | 10-07-2019 |
| | | JP   2016527264 A | 08-09-2016 |
| | | KR  20160038047 A | 06-04-2016 |
| | | LT      3027186 T | 10-09-2019 |
| | | PL      3027186 T3 | 31-12-2019 |
| | | PT      3027186 T | 09-09-2019 |

Pour tout renseignement concernant cette annexe : voir Journal Officiel de l'Office européen des brevets, No.12/82

EPO FORM P0460

page 4 de 5

**ANNEXE AU RAPPORT DE RECHERCHE EUROPEENNE
RELATIF A LA DEMANDE DE BREVET EUROPEEN NO.**

EP 19 20 9272

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

27-01-2021

| Document brevet cité au rapport de recherche | Date de publication | Membre(s) de la famille de brevet(s) | Date de publication |
|---|---|---|---|
| | | RU  2016106152 A | 31-08-2017 |
| | | SI    3027186 T1 | 30-09-2019 |
| | | US  2015038457 A1 | 05-02-2015 |
| | | US  2016166554 A1 | 16-06-2016 |
| | | US  2016325021 A1 | 10-11-2016 |
| | | US  2018021480 A1 | 25-01-2018 |
| | | US  2018169304 A1 | 21-06-2018 |
| | | US  2018318469 A1 | 08-11-2018 |
| | | US  2019184065 A1 | 20-06-2019 |
| | | US  2020390940 A1 | 17-12-2020 |
| | | WO  2015015407 A1 | 05-02-2015 |

EPO FORM P0460

Pour tout renseignement concernant cette annexe : voir Journal Officiel de l'Office européen des brevets, No.12/82

page 5 de 5

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005112888 A
- WO 2005067994 A
- WO 2010015901 A
- WO 2010052430 A
- WO 2012104419 A
- WO 2013186493 A
- FR 2979539
- CN 102805882
- KR 20140025117

- EP 2581079 A
- WO 2000046253 A
- WO 2009071697 A
- WO 2004092222 A
- FR 2983483
- FR 1352971
- WO 8600079 A

**Littérature non-brevet citée dans la description**

- **MICHAEL H GOLD.** *Clinical Interventions in Aging,* 2007, 369-376

- **CHO et al.** *Pak. J. Pharm. Sci.,* Janvier 2001, vol. 24 (1), 87-93